# EUROPEAN PATENT SPECIFICATION

(11) **EP 3 705 478 B1**
(45) Date of publication and mention of the grant of the patent: **04.10.2023**
(21) Application number: 18801601.8
(22) Date of filing: 15.05.2018
(51) Int. Cl.: C07D 403/04, C07D 471/04, A61K 31/53, A61P 35/00, C07D 471/10, C07D 487/04

(54) **TRIAZINE COMPOUND AND PHARMACEUTICALLY ACCEPTABLE SALT THEREOF**
TRIAZINVERBINDUNG UND DESSEN PHARMAZEUTISCH VERTRÄGLICHES SALZ
COMPOSÉ TRIAZINE ET SON SEL PHARMACEUTIQUEMENT ACCEPTABLE

(30) Priority: 15.05.2017 CN 201710339199
(43) Date of publication of application: 09.09.2020
(73) Proprietor: Shenzhen Forward Pharmaceuticals Co., Ltd., Shenzhen, Guangdong 518057 (CN)
(72) Inventor: TALLEY, J. John, Shenzhen, Guangdong 518057 (CN); YANG, Xuan, Shenzhen, Guangdong 518057 (CN); ZHU, Chenggang, Shenzhen, Guangdong 518057 (CN); XU, Liangliang, Shenzhen, Guangdong 518057 (CN)
(74) Representative: Lavoix
(86) International application number: PCT/CN2018/086929
(87) International publication number: WO 2018/210246

(56) References cited:
- EP-A1- 3 112 364
- EP-A1- 3 157 916
- EP-A1- 3 464 275
- CN-A- 105 175 349
- CN-A- 105 461 695
- CN-A- 106 132 957
- CN-A- 106 559 991
- CN-A- 106 928 150
- CN-A- 106 928 200
- US-A1- 2017 342 055

## Description

### TECHNICAL FIELD

The invention relates to a triazine compound for treating cancer and a pharmaceutically acceptable salt thereof, in particular to a compound for treating small cell lung cancer and a pharmaceutically acceptable salt thereof.

### BACKGROUND TECHNIQUE

In the field of anticancer drugs, there is always a need for new anticancer compounds with better activity/selectivity. EGFR is known to be a member of the transmembrane protein tyrosine kinase of the erbB receptor family. Homodimerization and/or heterodimerization of the erbB receptor lead to phosphorylation of certain tyrosine residues in the intracellular domain, and activate various intracellular signaling pathways involved in cell proliferation and survival. Dysregulation of erbB family signaling may lead to cell proliferation, invasion, metastasis, and angiogenesis, and has been reported in cancers such as lung cancer, head and neck cancer, and breast cancer. Therefore, as a target for the development of anticancer drugs, many drugs targeting EGFR or erbB2 are currently used in clinic. For example, reviews about erbB receptor signaling and its involvement in tumorigenesis are published in the New England Journal of Medicine, 2008, No. 358, pages 1160-1174, and Biochemical and Biophysical Research Communications, 2004, Vol. 319, pages 1-11. CN 106 132 957 refers to a 2-arylaminopyridine, pyrimidine or triazine derivative and a preparation method and application thereof.

However, there are EGFR activating mutations (such as L858R and delE746_A750) in practical cancer treatment, for example, see Science, 2004, No. 304, pages 1497-500, and New England Journal of Medicine, 2004, No. 350, pages 2129-39. As compared to wild-type (WT) EGFR, these most common EGFR activating mutations (L858R and delE746_A750) have increased affinity for small-molecule tyrosine kinase inhibitors (e.g., gefitinib and erlotinib), and decreased affinity for adenosine triphosphate (ATP). Finally, acquired resistance to gefitinib or erlotinib treatment is generated, for example, due to gatekeeper residue T790M mutation. Since this mutation results in resistance to existing drugs targeting EGFR, there is still a need in the art for a new compound inhibiting EGFR, including gatekeeper mutation. The new compound should have favorable activity and/or selectivity for wild-type EGFR, relative to EGFR in the form of an activating mutant (e.g., L858R EGFR mutant, or delE746_A750 mutant, or Exon19 deletion EGFR mutant), and/or EGFR in the form of a resistance mutant (e.g., T790M EGFR mutant). In other words, there is a need in the art for a compound exhibiting higher inhibition on certain forms of activated or resistant mutant EGFR while having relatively lower inhibition on wild-type EGFR, so as to not only exert anti-cancer efficacy, but also reduce adverse responses and toxicology associated with inhibition of wild-type EGFR (e.g., rash and/or diarrhea).

In order to solve this problem, after research the inventors have surprisingly discovered a class of anilino-triazine compounds that exhibit higher inhibition effect on the mutant form of EGFR, while having relatively lower inhibition on wild-type EGFR. In addition, these compounds have good pharmacological effects, acceptable toxicological effects, and favorable pharmacokinetic properties.

### CONTENTS OF THE INVENTION

The present invention provides a compound of formula (I) or a pharmaceutically acceptable salt thereof: 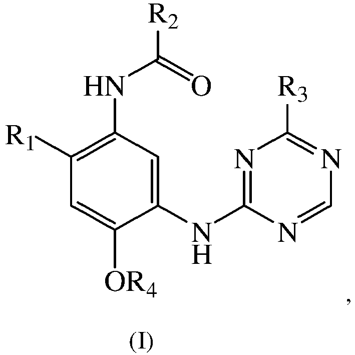
wherein R₁ is -NR₅R₆, and wherein R₅ and R₆ are each independently selected from C₁-C₆ alkyl, and wherein the alkyl is unsubstituted or substituted by -NR₇R₈;
wherein R₂ is selected from C₂-C₆ alkenyl, and the alkenyl is unsubstituted or substituted by -NR₇R₈;
wherein at each occurrence R₇ and R₈ are each independently selected from H or C₁-C₆ alkyl;
wherein R₃ is selected from: 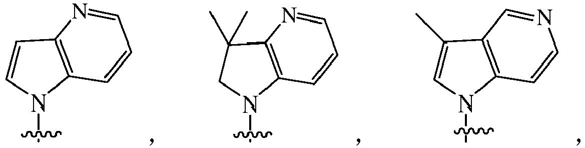 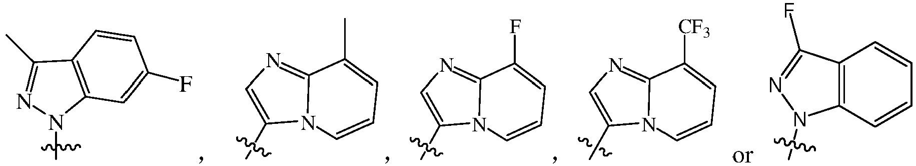 ; and
wherein R₄ is selected from C₁-C₆ alkyl.

According to an embodiment of the present invention, wherein the R₁ is selected from:

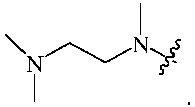

According to an embodiment of the present invention, wherein the R₂ is selected from:

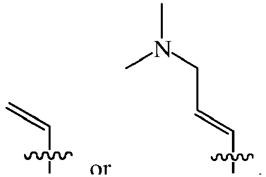

According to an embodiment of the present invention, wherein the R₃ is selected from:

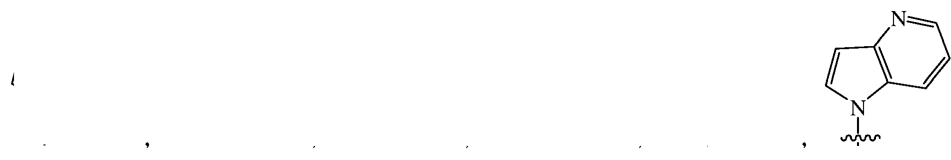

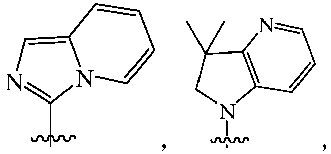

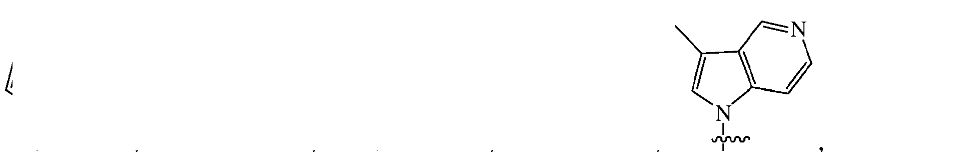

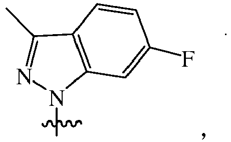

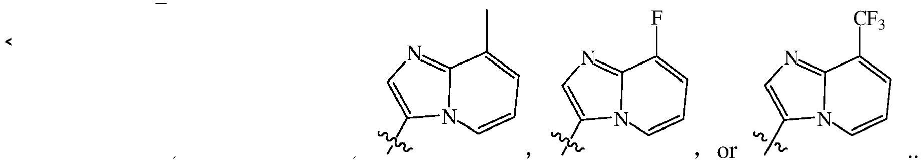

According to an embodiment of the present invention, wherein the compound is selected from:
N-(5-((4-(1H-pyrrolo[3,2-b]pyridin-1-yl)-1,3,5-triazin-2-yl)amino)-2-((2-(dimethylamino)ethy 1)(methyl)amino)-4-methoxyphenyl)acrylamide (Compound 6) 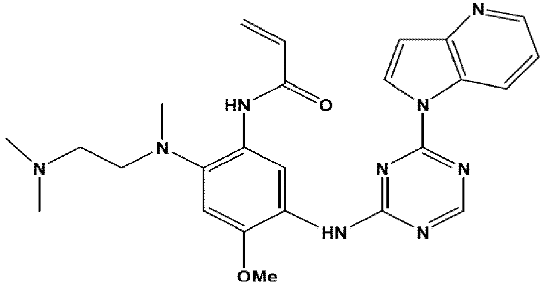
N-(5-((4-(3,3-dimethyl-2,3-dihydro-1H-pyrrolo[3,2-b]pyridin-1-yl)-1,3,5-triazin-2-yl)amino)-2 -((2-(dimethylamino)ethyl)(methyl)amino)-4-methoxyphenyl)acrylamide (Compound 11) 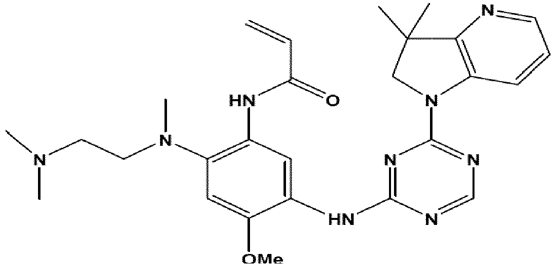
N-(2-((2-(dimethylamino)ethyl)(methyl)amino)-4-methoxy-5-((4-(3-methyl-1H-pyrrolo[3,2-c) pyridin-1-yl)-1,3,5-triazin-2-yl)amino)phenyl)acrylamide (Compound 18) 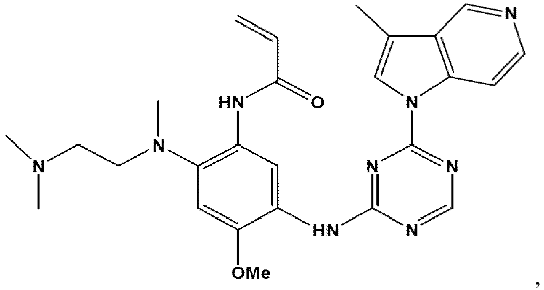
N-(2-((2-(dimethylamino)ethyl)(methyl)amino)-5-((4-(6-fluoro-3-methyl-1H-indazol-1-yl )-1, 3,5-triazin-2-yl)amino)-4-methoxyphenyl)acrylamide (Compound 20) 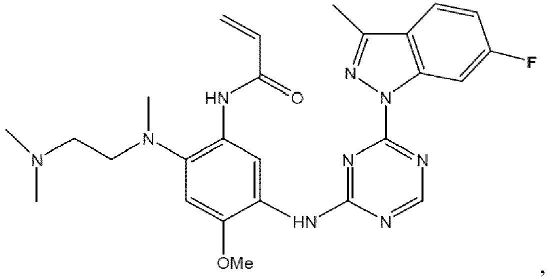
N-(2-((2-(dimethylamino)ethyl)(methyl)amino)-4-methoxy-5-((4-(8-methylimidazolo[1,2-a]py ridin-3-yl)-1,3,5-triazin-2-yl)amino)phenyl)acrylamide (Compound 26) 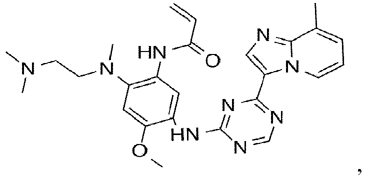
N-(2-((2-(dimethylamino)ethyl)(methyl)amino)-5-((4-(8-fluoroimidazolo[1,2-a]pyridin-3-yl)-1 ,3,5-triazin-2-yl)amino)-4-methoxyphenyl)acrylamide (Compound 27) 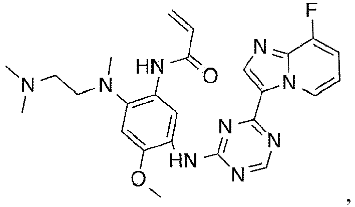
N-(2-((2-(dimethylamino)ethyl)(methyl)amino)-4-methoxy-5-((4-(8-(trifluoromethyl)imidazol o[1,2-a)pyridin-3-yl)-1,3,5-triazin-2-yl)amino)phenyl)acrylamide (Compound 28) 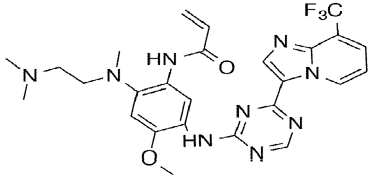 or
N-(2-((2-(dimethylamino)ethyl)(methyl)amino)-5-((4-(3-fluoro-1H-indazol-1-yl)-1,3,5-triazin-2-yl)amino)-4-methoxyphenyl)acrylamide (Compound 29)

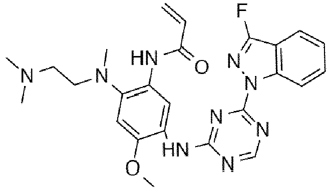

The invention also provides a pharmaceutical composition comprising the compound according to the invention or a pharmaceutically acceptable salt thereof.

The present invention also the compound of the present invention or a pharmaceutically acceptable salt thereof for use for treating small cell lung cancer.

### Definitions

In the present invention, the meanings of following terms are described as follows:
The "alkyl" in the present invention refers to a linear, branched saturated hydrocarbon group, preferably an alkyl with 12 or less carbon atoms, and more preferably an alkyl with 6 or less carbon atoms. Examples of alkyl include methyl, ethyl, n-propyl, isopropyl, cyclopropyl, n-butyl, isobutyl, tert-butyl, cyclobutyl, n-pentyl, isopentyl, neopentyl, cyclohexyl, n-hexyl, isohexyl, 2,2,-methylbutyl, and 2,3-dimethylbutyl. The "C₁₋₆ alkyl" of the present invention refers to a linear, branched saturated hydrocarbon group including 1-6 carbon atoms. The "C₁₋₃ alkyl" of the present invention refers to a linear, branched saturated hydrocarbon group including 1-3 carbon atoms.

The "alkenyl" in the present invention refers to a linear or branched unsaturated hydrocarbon group including one or more carbon-carbon double bonds (C=C). The "C₂₋₆ alkenyl" of the present invention refers to an unsaturated hydrocarbon group including 1 or 2 carbon-carbon double bonds and 2-6 carbon atoms. The "C₂₋₄ alkenyl" of the present invention refers to an unsaturated hydrocarbon group including 1 or 2 carbon-carbon double bonds and 2-4 carbon atoms. For example, vinyl, propenyl, n-butenyl, butadienyl, pentenyl, and hexenyl.

The "halogen" in the present invention refers to fluorine, chlorine, bromine, and iodine.

In the context of this specification, unless indicated to the contrary, the term "treatment" also includes "prevention". The term "treatment" as used herein is intended to have its ordinary meaning as follows: treating a disease to completely or partially relieve one, some, or all of its symptoms, or correcting or compensating for underlying pathology. The term "prevention" as used herein is intended to have its normal daily meaning, and includes primary prevention for preventing the development of the disease, and secondary prevention for preventing the occurrence of the disease, temporary or continuous prevention of the exacerbation or deterioration of the patient's disease or the occurrence of new symptoms related to diseases.

### Pharmaceutically Acceptable Salts

Those skilled in the art will understand that a salt of the compound of formula (I) including a pharmaceutically acceptable salt may be prepared. These salts may be prepared in situ during the final isolation and purification of the compound, or prepared by separately reacting the purified compound in a form of free acid or free base with a suitable base or acid, respectively.

A pharmaceutically acceptable acid addition salt may be formed together with inorganic and organic acids, for example, acetate, aspartate, benzoate, benzenesulfonate, bromide/hydrobromide, bicarbonate/carbonate, bisulfate/sulfate, camphorsulfonate, chloride/hydrochloride, citrate, ethanedisulfonate, fumarate, glucoheptonate, gluconate, glucuronate, hippurate, hydroiodide/iodide, isethionate, lactate, lactobionate, lauryl sulfate, malate, maleate, malonate, mandelate, methanesulfonate, methylsulfate, naphthoate, naphthalene sulfonate, nicotinate, nitrate, stearate, oleate, oxalate, palmitate, pamoate, phosphate/hydrogen phosphate/dihydrogen phosphate, polygalacturonate, propionate, stearate, succinate, sulfosalicylate, tartrate, tosylate, and trifluoroacetate.

Inorganic acids that may be used to form salts include, for example, hydrochloric acid, hydrobromic acid, sulfuric acid, nitric acid, phosphoric acid, and the like.

Organic acids that may be used to form salts include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, etc.. A pharmaceutically acceptable base addition salt may be formed with an inorganic or organic base.

Inorganic bases that may be used to form salts include, for example, ammonium salts and metals of Groups I-XII of the Periodic Table of the Elements. In some embodiments, the salts are derived from sodium, potassium, ammonium, calcium, magnesium, iron, silver, zinc, and copper; particularly suitable salts include salts of ammonium, potassium, sodium, calcium, and magnesium.

Organic bases that may be used to form salts include, for example, primary amines, secondary amines and tertiary amines. Substituted amines include naturally occurring substituted amines, cyclic amines, alkali ion exchange resins, and the like. Some organic amines include isopropylamine, diethanolamine, diethylamine, lysine, meglumine, piperazine, and tromethamine.

The pharmaceutically acceptable salts of the present invention may be synthesized from basic or acidic moieties by conventional chemical methods. Generally, these salts may be prepared by reacting the free acid form of these compounds with a stoichiometric amount of a suitable base (hydroxide, carbonate, bicarbonate, etc. of Na, Ca, Mg, or K), alternatively be prepared by reacting the free base form of these compounds with a stoichiometric amount of a suitable acid. These reactions are usually carried out in water or in an organic solvent, or in a mixture of both. Generally, when appropriate, a non-aqueous medium such as diethyl ether, ethyl acetate, ethanol, isopropanol, or acetonitrile is used. A list of other suitable salts may be found in "Remington's Pharmaceutical Sciences", 20th edition, Mack Publishing Company, Easton, Pa., (1985); and Stahl and Wermuth, "Handbook of Pharmaceutical Salts: Properties, Selection, and Use" (Wiley-VCH, Weinheim, Germany, 2002).

Solvates of the compounds of formula (I), including pharmaceutically acceptable solvates, may also be prepared. "Solvate" refers to a complex of variable stoichiometry formed by a solute and a solvent. Such solvents for the purposes of the present invention do not affect the biological activity of the solutes. Examples of suitable solvents include, but are not limited to water, MeOH, EtOH, and AcOH. The solvate in which water is a solvent molecule usually refers to a hydrate. Hydrates include components that contain stoichiometric amounts of water, as well as components that contain variable amounts of water.

As used herein, the term "pharmaceutically acceptable" means a compound suitable for pharmaceutical use. Salts and solvates (e.g., hydrates and salt hydrates) of the compounds of the present invention suitable for use in medicine are those in which the counter-ions or binding solvents are pharmaceutically acceptable. However, salts and solvates with non-pharmaceutically acceptable counter-ions or binding solvents are also included within the scope of the present invention, for example, as intermediates for the preparation of other compounds of the present invention and the pharmaceutically acceptable salts and solvates thereof.

The compound of formula (I) (including the salt and solvate thereof) may exist in a crystalline form, an amorphous form, or a mixture thereof. The compound or the salt or solvate thereof may also exhibit polymorphism, i.e., the ability to appear in different crystalline forms. These different crystalline forms are generally known as "polymorphs". Polymorphs have the same chemical composition, but the packing, geometric arrangement, and other description characteristics of the crystalline solid state are different. Therefore, polymorphs may have different physical properties, such as shape, density, hardness, deformability, stability, and solubility properties. Polymorphs usually exhibit different melting points, IR spectra, and X-ray powder diffraction patterns, all of which may be used for identification. Those skilled in the art will understand, for example, that different polymorphic forms may be produced by changing or adjusting the conditions used in the crystallization/recrystallization of the compound of formula (I).

The present invention also includes different isomers of the compound of formula (I). "Isomer" refers to compounds that have the same composition and molecular weight, but differ in physical and/or chemical properties. The difference in structure may be in the structure (geometric isomers) or in the ability to rotate plane polarized light (stereoisomers). With regard to stereoisomers, the compound of formula (I) may have one or more asymmetric carbon atoms, and may occur as racemates, a racemic mixture, and as individual enantiomers or diastereomers. All such isomeric forms are included within the scope of the present invention, including a mixture thereof. If the compound contains a double bond, the substituent may be in E or Z configuration. If the compound contains a disubstituted cycloalkyl, the substituent of the cycloalkyl may have a cis- or trans-configuration. It is also desirable to include all tautomeric forms.

Any asymmetric atom (such as carbon, etc.) of the compound of formula (I) may exist in racemic or enantiomeric enrichment, such as (R)-, (S)-, or (R,S)-configuration. In some embodiments, in the (R)- or (S)-configuration, each asymmetric atom has an enantiomeric excess of at least 50%, an enantiomeric excess of at least 60%, an enantiomeric excess of at least 70%, an enantiomeric excess of at least 80%, an enantiomeric excess of at least 90%, an enantiomeric excess of at least 95%, or an enantiomeric excess of at least 99%. If possible, the substituents on the atoms with unsaturated double bonds exist in the form of cis- (Z)-or trans- (E)-.

Thus, as used herein, the compound of formula (I) may be in the form of any one of the possible isomer, rotamer, atropisomer, tautomer, or a mixture thereof, for example, as a substantially pure geometrical isomer (cis or trans), diastereomer, optical isomer (enantiomers), racemates or a mixture thereof.

Any resulting mixture of isomers may be separated into pure or substantially pure geometric or optical isomers, diastereomers, racemates, for example, by chromatography and/or fractional crystallization, on the basis of the physicochemical differences of the components.

Any resulting racemate of the final product or intermediate may be resolved into optical rotation enantiomers by known methods (e.g., by separation of its diastereomeric salts), it is obtained by using optically active acid or base, and optically active acidic or basic compound is released. In particular, the basic moiety may therefore be used to resolve the compound of the invention into its optical rotation enantiomers, for example, by fraction crystallization of salts formed with optically active acids (e.g., tartaric acid, dibenzoyltartaric acid, diacetyltartaric acid, di-O,O'-p-toluoyltartaric acid, mandelic acid, malic acid or camphor-10-sulfonic acid). Racemic products may also be resolved by chiral chromatography, such as a high-pressure liquid chromatography (HPLC) using chiral adsorbents.

The invention includes unlabeled form as well as isotopically labeled form of the compound of formula (I). An isotopically labeled compound has a structure described by the chemical formula given herein, except that one or more atoms are replaced with atoms having a selected atomic weight or mass number. Examples of isotopes that may be incorporated into the compound of the present invention include isotopes of hydrogen, carbon, nitrogen, oxygen, phosphorus, fluorine, and chlorine, such as ²H, ³H, ¹¹C, ¹³C, ¹⁴C, ¹⁵N, ¹⁸F , ³¹P, ³²P, ³⁵S, ³⁶Cl, ¹²⁵I. The present invention includes various isotopically-labeled compounds as defined herein, such as those in which radioisotopes (e.g., ³H and ¹⁴C) appear or those in which non-radioactive isotopes (e.g., ²H and ¹³C) appear. These isotope-labeled compounds may be used for metabolic studies (e.g., using ¹⁴C), reaction kinetic studies (e.g., using ²H or ¹³C), detection or imaging techniques, such as positron emission tomography (PET) or single photon emission computed tomography (SPECT), including tissue distribution analysis of drug substrates, or radiation therapy for patients. In particular, ¹⁸F or labeled compounds may be particularly needed for PET or SPECT studies. A isotopically-labeled compound of formula (I) may usually be prepared by conventional techniques known to those skilled in the art, or by methods similar to those described in the accompanying Examples and Preparation Examples, while replacing the previous unlabeled reagents with appropriate isotopically-labeled reagents.

In addition, substitution with heavier isotopes, especially deuterium (i.e., ²H or D) may bring certain therapeutic advantages due to stronger metabolic stability, such as increased half-life in the body or reduced dose requirements or improvement of therapeutic index. It is understood that deuterium is considered herein as a substituent of the compound of formula (I). The concentration of the heavier isotope, especially deuterium, may be determined by the isotopic enrichment factor. The term "isotopic enrichment factor" as used herein refers to the ratio between the isotopic abundance and the natural abundance of a specific isotope. If the substituent in the compound of the present invention is labeled as deuterium, then for each deuterium atom marked, the compound has an isotopic enrichment factor of at least 3500 (52.5% deuterium incorporation at each deuterium atom marked), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

### Pharmaceutical Composition

The present invention provides a pharmaceutical composition comprising the compound of formula (I) or a pharmaceutically acceptable salt thereof as described above and a pharmaceutically acceptable diluent or carrier.

The pharmaceutical composition may be formulated for a specific route of administration, such as oral administration, parenteral administration, rectal administration, and the like. Furthermore, the pharmaceutical composition according to the present invention may be prepared in solid form (including but not limited to capsules, tablets, pills, granules, powders or suppositories), or in liquid form (including but not limited to solutions, suspensions or emulsions). The pharmaceutical composition may undergo conventional pharmaceutical operations (e.g., sterilization) and/or may contain conventional inert diluents, lubricants or buffers, and auxiliary materials such as preservatives, stabilizers, wetting agents, emulsifiers and buffers, etc..

Generally, the pharmaceutical composition is a tablet or gelatin capsule, which contains an active ingredient and
a) a diluent, such as lactose, dextrose, sucrose, mannitol, sorbitol, cellulose and/or glycine;
b) a lubricant, such as silica, talc, stearic acid and its magnesium or calcium salt, and/or polyethylene glycol; for a tablet, it also includes:
c) a binder, such as magnesium aluminum silicate, starch paste, gelatin, tragacanth, methyl cellulose, sodium carboxymethyl cellulose, and/or polyvinylpyrrolidone; if necessary, it may also include
d) a disintegrant, such as starch, agar, alginic acid or its sodium salt, or an effervescent mixture; and/or
e) an absorbent, colorant, flavoring agent and sweetener.

According to methods known in the art, tablets may be film-coated or enteric-coated.

Suitable compositions for oral administration include an effective amount of a compound of formula (I) or a pharmaceutically acceptable salt thereof, which is in the form of a tablet, lozenge, water or oil suspension, dispersible powder or granule, emulsion, hard or soft capsule, or a syrup or elixir. A composition for oral use is prepared according to any method known in the art for preparing a pharmaceutical composition, and in order to provide a refined and palatable formulation, the composition may contain one or more agents selected from sweetener, flavoring agent, colorant and preservative. A tablet may contain an active ingredient in admixture with a non-toxic pharmaceutically acceptable excipient which is suitable for the manufacture of the tablet. These excipients are, for example, inert diluents (such as calcium carbonate, sodium carbonate, lactose, calcium phosphate, or sodium phosphate); granulating agents and disintegrating agents (such as corn starch, or alginic acid); binders (such as starch , gelatin or arabic gum); and lubricants (such as magnesium stearate, stearic acid or talc). A tablet is uncoated or coated by known techniques to delay disintegration and absorption in the gastrointestinal tract, thereby providing a long-lasting effect over a longer period. For example, a time delay material may be used, such as glyceryl monostearate or glyceryl distearate. A formulation for oral administration may be presented in hard gelatin capsule, wherein the active ingredient is mixed with an inert solid diluent (such as calcium carbonate, calcium phosphate, or kaolin), or in soft gelatin capsule, wherein the active ingredient is mixed with water or an oil medium (such as peanut oil, liquid paraffin or olive oil).

Some injectable compositions are isotonic aqueous solutions or suspensions, and suppositories are advantageously prepared from fatty milk or suspensions. The composition may be sterilized and/or contain auxiliary materials, such as preservatives, stabilizers, wetting or emulsifying agents, dissolution promoters, salts for adjusting osmotic pressure, and/or buffering agents. In addition, it may also contain other therapeutically valuable substances. The compositions are prepared according to conventional mixing, granulating or coating methods respectively, and contain about 0.1-75%, or about 1-50% of the active ingredient.

Since water may promote the degradation of certain compounds, the present invention also provides an anhydrous pharmaceutical composition and dosage form, which contains the compound of the present invention as an active ingredient.

The anhydrous pharmaceutical composition and dosage form according to the present invention may be prepared by using anhydrous or low water content ingredients and low water content or low humidity conditions. An anhydrous pharmaceutical composition may be prepared and stored in order to maintain the anhydrous nature. Therefore, the anhydrous composition is packaged by using materials known to prevent contact with water so that it may be contained in a suitable formulation kit. Examples of suitable packaging include, but not limited to, airtight foil, plastic, unit-dose containers (e.g., vials), blister packs, and tape packs.

The present invention further provides a pharmaceutical composition and dosage form comprising one or more agents that reduce the decomposition rate of the compound of the present invention as an active ingredient. The agent (which is referred to herein as a "stabilizer") includes, but not limited to, antioxidants (e.g., ascorbic acid), pH buffers, or salt buffers, and the like.

For an individual with about 50-70 kg of body weight, a unit dose of the pharmaceutical composition or combination product according to the present invention may have about 1-1000 mg of active ingredient, or about 1-500 mg, or about 1-250 mg, or about 1-150 mg, or about 0.5-100 mg, or about 1-50mg of active ingredient. The therapeutically effective dose of a compound, pharmaceutical composition, or the combination product depends on the species, body weight, age, and condition of the individual, the condition or disease being treated, or the severity thereof. A general practitioner, clinician, or veterinarian may easily determine the effective amount of each active ingredient required to prevent, treat, or inhibit the development of a disorder or disease.

### Therapeutic Use

In another aspect, the invention provides a compound of formula (I) or a pharmaceutically acceptable salt thereof for use as a medicament. In another aspect, the present invention also provides the compound of formula (I) or a pharmaceutically acceptable salt thereof for use for treating non-small cell lung cancer.

On the other hand, the present invention also provides for producing an anticancer effect in a warm-blooded animal such as a human in need of such treatment, comprising: administering an effective amount of a compound of formula (I) or a pharmaceutical acceptable salt thereof to said animal.

In another aspect, the present invention also provides for a compound of formula (I) or a pharmaceutically acceptable salt thereof and another anti-tumor substance to be administrated simultaneously, independently or sequentially for the treatment of cancer.

In any of the aspects or embodiments mentioned above, the cancer is non-small cell lung cancer.

The above-mentioned compound of formula (I) or a pharmaceutically acceptable salt thereof may be used as a single therapy, alternatively, the treatment may also involve conventional surgery, or radiotherapy, or chemotherapy, or immunotherapy in addition to the compound of the present invention. Such chemotherapy and the compounds of the present invention may be administered side-by-side, simultaneously, sequentially, or separately, and may contain one or more of the following types of anti-tumor agents:
(i) antiproliferative/antitumor drugs and their combinations used in medical oncology, such as alkylating agents (e.g., cisplatin, oxaliplatin, carboplatin, cyclophosphamide, nitrogen mustard, melphalan, chlorambucil, busulfan, temozolomide, nitrosourea); antimetabolites (such as gemcitabine, and antifolates, such as fluoropyrimidines (e.g., 5-fluorouracil and tegafur), raltitrexed, methotrexate, cytarabine, hydroxyurea); antitumor antibiotics (e.g., anthracyclines such as doxorubicin, bleomycin, doxorubicin, daunorubicin, epirubicin, idarubicin, mitomycin C, actinomycin, mithramycin); antimitotic agents (e.g., vinca alkaloids, such as vincristine, vinblastine, vindesine, vinorelbine; and taxanes such as paclitaxel, taxotere); topoisomerase inhibitors (such as epipodophyllotoxin (e.g., etoposide, teniposide), amsacrine, topotecan, camptothecin);
(ii) cell growth inhibitors, such as anti-estrogen drugs (e.g., tamoxifen, fulvestrant, toremifene, raloxifene, and droloxifene), anti-androgen drugs (e.g., bicalutamide, flutamide, nilutamide, cyproterone acetate), LHRH antagonists or LHRH agonists (e.g. goserelin, leuprolide, and buserelin), progestins (e.g. megestrol acetate), aromatase inhibitors (e.g., anastrozole, letrozole, exemestane), 5α-reductase inhibitors (e.g., finasteride);
(iii) anti-invasive agents, e.g., c-Src kinase family inhibitors, such as dasatinib and bosutinib (SKI-606), and metalloproteinase inhibitors (such as marimastat), inhibitors of urokinase plasminogen activator receptor function, or antibodies to heparanase;
(iv) inhibitors of growth factor function: for example, such inhibitors include growth factor antibodies and growth factor receptor antibodies (e.g., anti-erbB2 antibody trastuzumab, anti-EGFR antibody panitumumab, anti-erbB1 antibody cetuximab and any growth factor or growth factor receptor antibody); such inhibitors also include: tyrosine kinase inhibitors, such as inhibitors of the epidermal growth factor family (such as EGFR family tyrosine kinase inhibitors, such as N-(3-chloro-4-fluorophenyl)-7-methoxy-6-(3-morpholinylpropoxy)-quinazolin-4-amine (gefitinib, ZD1839), N-(3-ethynylphenyl)-6,7-bis(2-methoxyethoxy) quinazolin-4-amine (erlotinib, OSI-774), 6-acrylamido-N-(3-chloro-4-fluorophenyl)-7-(3-morpholinylpropoxy)-quinazolin-4-amine (CI 1033), erbB2 tyrosine kinase inhibitor (e.g. lapatinib); inhibitors of the hepatocyte growth factor family; inhibitors of insulin growth factor family; inhibitors of platelet-derived growth factor family, such as imatinib and/or nilotinib (AMN107); serine/threonine kinase inhibitors (e.g., Ras/Raf signaling inhibitors, such as farnesyl transferase inhibitors, such as sorafenib (BAY43-9006), tipifarnib (R115777), lonafarnib (SCH66336)), cell signaling inhibitors via mEK and/or AKT kinase , C-kit inhibitors, abl kinase inhibitors, PI3 kinase inhibitors, Plt3 kinase inhibitors, CSF-1R kinase inhibitors, IGF receptor (insulin-like growth factor) kinase inhibitors; aurora kinase inhibitors (e.g., AZD1152, PH739358, VX-680, MLN8054, R763, MP235, MP529, VX-528, AX39459), cyclin-dependent kinase inhibitors, such as CDK2 and/or CDK4 inhibitors;
(v) anti-angiogenic agents, e.g., agents that inhibit the action of vascular endothelial growth factor, such as anti-human vascular endothelial cell growth factor antibody bevacizumab and, for example, VEGF receptor tyrosine kinase inhibitors, such as vandetanib (ZD6474), vatalanib (PTK787), sunitinib (SU11248), axitinib (AG-013736), pazopanib (GW786034), 4-(4-fluoro-2-methylindol-5-yloxy)-6-methoxy-7-(3-pyrrolidin-1-ylpropoxy)quinazoline (AZD2171; Example 240 in WO 00/47212), for example, those disclosed in WO 97/22596, WO 97/30035, WO 97/32856 and WO 98/13354;
(vi) vascular damaging agents, such as Combretastatin A4 and the compounds disclosed in WO 99/02166, WO 00/40529, WO 00/41669, WO 01/92224, WO 02/04434 and WO 02/08213;
(vii) endothelin receptor antagonists, such as zibotentan (ZD4054) or atrasentan;
(viii) gene therapy methods, including, for example, methods for replacing abnormal genes (such as abnormal p53 or abnormal BRCA1 or BRCA2); GDEPT (gene-directed enzyme prodrug therapy) methods, such as those using cytosine deaminase, thymidine kinase, or bacterial nitroreductase; methods for increasing patients' tolerance to chemotherapy or radiation therapy, such as multidrug resistance gene therapy; and
(ix) immunotherapy methods, including, for example, in vitro and in vivo methods for increasing the immunogenicity of patients' tumor cells, such as transfection with cytokines (e.g., interleukin 2, interleukin 4, or granulocyte macrophage colony-stimulating factor); methods for reducing T cell inefficiency; methods using transfected immune cells (such as cytokine-transfected dendritic cells); methods using cytokine-transfected tumor cell lines; methods using anti-idiotypic antibodies; methods for reducing the function of immunosuppressive cells (e.g., regulatory T cells, myeloid-derived suppressor cells, or dendritic cells expressing IDO (indolamine 2,3-deoxygenase)); and methods using cancer vaccine consisting of proteins or peptides derived from tumor-related antigens (e.g., NY-ESO-1, mAGE-3, WT1, or Her2/neu).

Herein, if the term "combination therapy" is used to describe a combined treatment, it should be understood that this may mean simultaneous administration, independent administration, or sequential administration. The "combination administration" should be understood similarly. In one aspect of the invention, "combination therapy" refers to simultaneous administration. In another aspect of the invention, "combination therapy" refers to independent administration. In another aspect of the invention, "combination therapy" refers to sequential administration. When administered sequentially or independently, the delay in the administration of the second component should not, for example, lose the benefit of the effect produced by using the combination administration.

### Synthetic Method of the Compound according to the Present Invention

The compound of formula (I) according to the present invention may generally be synthesized according to the following scheme:

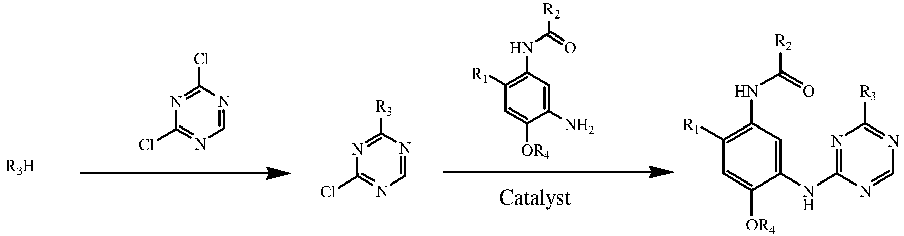

In the above process, the starting material 2,4-dichloro-1,3,5-triazine is coupled with a suitable fused ring compound R₃H to obtain an intermediate 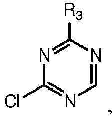 which reacts with the intermediate 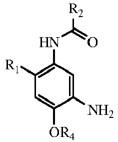 in the presence of catalyst to obtain the compound of formula (I), wherein R₃ is defined as above.

In addition, the synthetic route for preparation of the intermediate 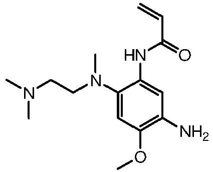 is as follows:

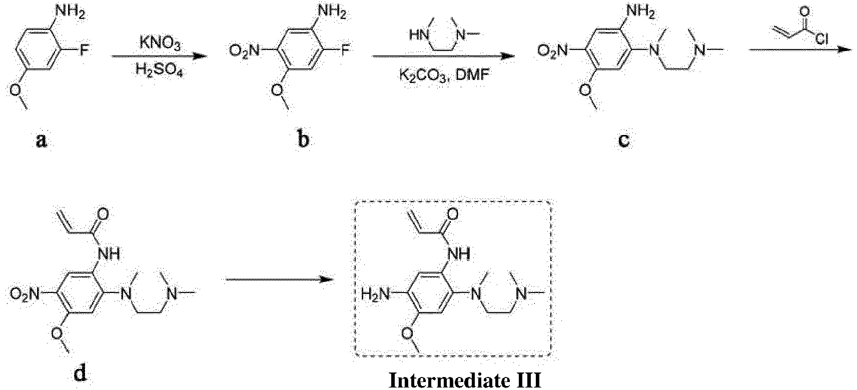

### SPECIFIC EMBODIMENTS

The method of the present invention is further described by the following examples.

### Synthesis of Intermediate III:

KNOs (35.8 g, 354 mmol) is slowly added to a solution of Compound a (50.0 g, 354 mmol) in H₂SO₄ (260 mL) at 0°C with stirring, and then stirring for 2 hours. TLC (petroleum ether: ethyl acetate = 5:1, Rf = 0.57) detection indicates complete conversion of the reactants. The reaction mixture is poured into ice water (3 kg), the pH is adjusted to 7 with aqueous NaOH solution, then extracting with ethyl acetate (1.5 Lx 2). The extracted organic phase is dried over Na₂SO₄, and concentrated to obtain a crude product. The crude product is purified by a silica gel column (the eluent is: petroleum ether: ethyl acetate = 20: 1-5: 1) to obtain Compound b(91.0 g, yield: 69%) as a yellow solid. **¹H NMR:**(ES6131-2-P1A, CDCl₃, 400MHz)*δ*7.45-7.43 (d, *J* = 9.2 Hz, 1H), 6.82-6.79 (d, *J* = 12.0 Hz, 1H), 3.89 (s, 3H), 3.69 (br s, 2H).

The mixture of Compound **b** (79.0g, 424mmol), N,N',N'-trimethylethane-1,2-diamine (65.1g, 637mmol, 82mL) and potassium carbonate (88.0g, 637mmol) in DMF ( 150 mL) is stirred at 90°C for 25 hours. TLC (petroleum ether: ethyl acetate = 3: 1) detection indicates complete consumption of the reactants. Water (1.5 L) is added to the reaction solution, extracting with dichloromethane (800 mL × 3), washing with saturated salt water(1 L), drying over Na₂SO₄, and concentrating to obtain a crude product. The crude product is purified by a silica gel column (the eluent is: dichloromethane: methanol = 100: 1-20: 1) to obtain Compound **c** (66.0 g, yield: 58%) as a yellow oil.

**¹H NMR:**(ES6131-2-P1A, CDCl₃, 400MHz)δ =7.31 (s, 1H), 6.63 (s, 1H), 3.90 (s, 3H), 3.06-3.03 (t, *J* = 6.0 Hz, 2H), 2.79 (s, 3H), 2.51-2.48 (t, *J* = 6.0 Hz, 2H), 2.31 (s, 6H).**LCMS:** 269.1 [M+H]⁺, Rt = 1.240.

Triethylamine (18.0 g, 178 mmol, 25 mL) is added to a solution of Compound c (18.0 g, 67.1 mmol) in dichloromethane (200 mL) at 0°C, then dropwise adding acryloyl chloride (8.0 g, 88.6 mmol, 7.2 mL). The reaction mixture is stirred at 0°C for 16 hours. TLC (dichloromethane: methanol = 20: 1) detection indicates almost complete reaction of the reactants. The reaction mixture is quenched with water (400 mL), then extracting with dichloromethane (200 mLx3). The organic phase is dried with Na₂SO₄, filtering and concentrating to give a crude product. The crude product is purified by silica gel column chromatography (dichloromethane: methanol = 100: 1-20: 1) to obtain Compound **d** (8.10 g, yield: 39%) as a brown oil.

**¹H NMR:**(ES6131-4-p1a, CDCl₃, 400MHz)*δ* = 10.12 (s, 1H), 9.19 (s, 1H), 6.79 (s, 1H), 6.49-6.44 (d, *J* = 17.2 Hz, 1H), 6.32-6.25 (dd, *J₁* = 10.0 Hz, *J₂* = 16.8 Hz , 2H), 5.75-5.72 (d, *J*= 10.0 Hz), 3.93 (s, 3H), 2.92-2.90 (t, *J* = 5.2 Hz, 2H), 2.79 (s, 3H), 2.49-2.46 (m, 2H), 2.33 (s, 6H). **LCMS:** 323.2 [M+H]⁺.

Ethanol (100 mL) and water (50 mL) are added to a mixture of Compound **d** (9.0 g, 27.9 mmol), Fe (7.8 g, 139 mmol) and NH₄Cl (7.5 g, 140 mmol), then stirring at 50°C for 16 hours. TLC (dichloromethane: methanol = 10: 1) detection indicates complete reaction of the reactants. The reaction solution is filtered and concentrated to obtain a crude product. The crude product is purified by silica gel column chromatography (dichloromethane: methanol = 20: 1), and the eluent is concentrated to obtain Intermediate **III** (7.6 g, yield: 93%) as an off-white solid. **¹H NMR:**(ES6131-12-pla, CDCl₃, 400MHz)*δ*7.08 (br s, 1H), 6.85 (s, 1H), 6.65-6.58 (dd, *J₁* = 10.0 Hz, *J* = 16.8 Hz, 1H), 6.45-6.41 (m, 1H), 5.86-5.83 (dd, *J₁* = 10.0 Hz, *J* = 16.8 Hz, 1H), 3.93 (s, 3H), 3.37-3.31(m, 2H), 3.08 (s, 2H), 2.75 (s, 6H), 2.68 (s, 3H).

### Example 1

### N-(2-((2-(dimethylamino)ethyl)(methyl)amino)-4-methoxy-5-((4-(3-methyl-1H-indol-1-yl)-1,3 ,5-triazin-2-yl)amino)phenyl)acrylamide (Compound 4) (not part of the present invention)

### Synthetic Route:

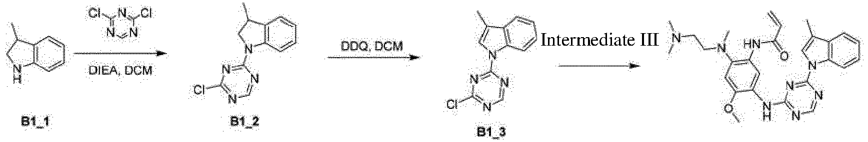

2,4-dichloro-1,3,5-triazine (562 mg, 3.75 mmol) is added at 0°C to a solution of Compound B1_1 (500 mg, 3.75 mmol) and diisopropylethylamine (533 mg, 4.13 mmol) in methylene chloride (3 mL), then stirring for 1 hour. TLC (petroleum ether: ethyl acetate = 10: 1, Rf = 0.48) detection indicates complete reaction of the reactants. The reaction mixture is concentrated to obtain a crude product. The crude product is purified by silica gel column chromatography (petroleum ether: ethyl acetate = 100: 1-25: 1) to obtain **Compound B1_2** (720 mg, yield: 70%) as an off-white solid.

**¹H NMR:**(ES6208-30-P1A , CDCl₃, 400MHz)*δ*8.51 (s, 0.5H), 8.47 (s, 0.5H), 8.3 (d, *J* = 8.0 Hz, 0.5H), 8.27 (d, *J* = 8.0 Hz, 0.5H), 7.26-7.15 (m, 2H), 7.06 (t, *J* = 7.6 Hz, 1H), 4.39 (t, *J* = 12.0 Hz, 1H), 3.74-3.70 (m, 1H), 3.50-3.42 (m, 1H), 1.33 (d, *J* = 6.8 Hz, 3H).

DDQ (1.1 g, 4.9 mmol) is added to a solution of Compound **B1_2** (600 mg, 2.4 mmol) in dichloromethane (15 mL). The reaction solution is stirred at 25°C for 5 hours under the protection of nitrogen. TLC (petroleum ether: ethyl acetate = 10: 1) detection indicates complete reaction of the reactants. The reaction solution is purified by silica gel column chromatography (petroleum ether: ethyl acetate = 25: 1) to obtain **Compound B1_3** (560 mg, yield: 94%) as a white solid.

**¹H NMR:**(ES6176-26-PlA,CDCl₃, 400MHz)*δ*8.81 (br s, 1H), 8.69-8.67 (d, *J*= 9.2 Hz, 1H), 7.91 (s, 1H), 7.56-7.54 (d, *J* = 8.0 Hz, 1H), 7.43-7.39 (t, *J* = 7.6 Hz, 1H), 7.36-7.33 (t, *J* = 7.6 Hz, 1H), 2.34 (s, 3H).

Compound **B1_3** (200 mg, 0.82 mmol) is added to a solution of Intermediate **III** (239 mg, 0.82 mmol) in tetrahydrofuran (10 mL), then adding pyridine (129 mg, 1.6 mmol). The reaction mixture is stirred at 25°C for 6 hours under the protection of nitrogen. TLC (petroleum ether: ethyl acetate = 5: 1) detection indicates complete reaction of the reactants. The reaction mixture is concentrated to obtain a crude product. The crude product is purified by prep-HPLC (column: Waters Xbridge 150*25 5u; mobile phase: [water(10mM NH₄HCO₃)-ACN]; B%: 48%-78%, 8min) to give **Compound 4** (79 mg, yield: 19%) as a yellow solid.

**¹H NMR:**(ES6176-27-P1A,CDCl₃, 400MHz)*δ*10.13 (s, 1H), 9.81-9.76 (br s, 1H), 8.79-8.77 (d, *J =* 8.4 Hz, 1H), 8.63 (s, 1H), 7.92-7.88 (br s, 1H), 7.54-7.52 (d, *J*= 7.6 Hz, 1H), 7.35-7.24 (m, 3H), 6.82 (s, 1H), 6.55-6.51 (m, 1H), 6.41-6.36 (m, 1H), 5.75-5.72 (d, *J =* 10.4 Hz, 1H), 3.90 (s, 3H), 2.91 (s, 2H), 2.73 (s, 3H), 2.49 (s, 3H), 2.31 (s, 8H).**LCMS:** 501.4 [M+H]⁺, Rt = 0.771. **HPLC:** 96.41% purity (220 nm), Rt = 5.06.

### Example 2

### N-(5-((4-(1H-pyrrolo[3,2-b]pyridin-1-yl)-1,3,5-triazin-2-yl)amino)-2-((2-(dimethylamino)ethy l)(methyl)amino)-4-methoxyphenyl)acrylamide (Compound 6)

### Synthetic Route:

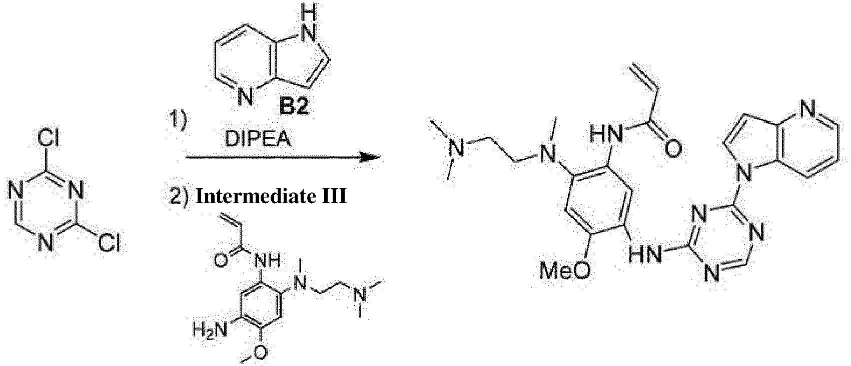

Compound **B2** (202 mg, 1.70 mmol) and diisopropylethylamine (234 mg, 1.80 mmol) are added to a solution of 2,4-dichloro-1,3,5-triazine (257 mg, 1.70 mmol) in DMF (12 mL) at 0°C, then stirring for 0.5 hour. Intermediate **III** (500 mg, 1.70 mmol) and diisopropylethylamine (234 mg, 1.80 mmol) are sequentially added to the reaction solution at 0°C. The reaction mixture is stirred at 25°C for 20 hours. TLC (petroleum ether: ethyl acetate = 1: 2, Rf = 0.35) detection indicates complete reaction of the reactants. The reaction mixture is purified by prep-HPLC (column: Waters Xbridge 150*25 5u; mobile phase: [water(10mM NH₄HCO₃)-ACN]; B%: 18%-48%, 10min) to give **Compound 6** (37.5mg , yield: 4.5%) as a bright yellow solid.

**¹H NMR:**(ES6045-14-P1A, CDCl₃, 400MHz)*δ*10.21 (br s, 1H), 9.01 (d, *J =* 8.0 Hz, 1H), 8.65 (s, 1H), 8.52 (d, *J =* 2.8 Hz, 1H), 7.87 (s, 1H), 7.20-7.28 (m, 1H), 6.95 (s, 1H), 6.84 (s, 1H), 6.50 (d, *J =* 16.0 Hz, 1H), 6.37-6.33 (m, 1H), 5.75 (d, *J =* 10.0 Hz, 1H), 3.92 (s, 3H), 2.91 (s, 2H), 2.75 (s, 3H), 2.33 (d, *J =* 4.0 Hz, 2H), 2.18 (s. 6H).

**LCMS:** 488.1 [M+H]⁺, Rt = 3.615. **HPLC:** 98.31% purity (220 nm), Rt = 3.52.

### Example 3

### N-(5-((4-(1H-benzo[d]imidazol-1-yl)-1,3,5-triazin-2-yl)amino)-2-((2-(dimethylamino)ethyl)(m ethyl)amino)-4-methoxyphenyl)acrylamide (Compound 8) (not part of the present invention)

### Synthetic Route:

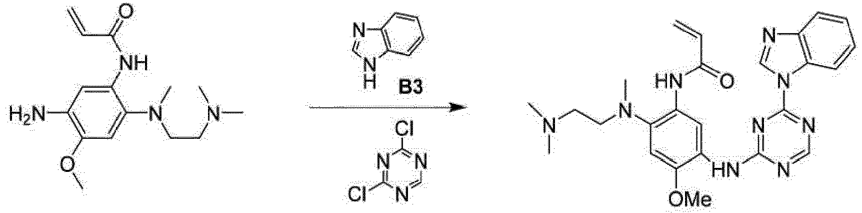

Compound **B3** (202 mg, 1.70 mmol) and diisopropylethylamine (234 mg, 1.80 mmol) are added to a solution of 2,4-dichloro-1,3,5-triazine (257 mg, 1.70 mmol) in DMF (8 mL) at 0°C, then stirring for 1 hour. Diisopropylethylamine (234 mg, 1.80 mmol) and Intermediate III (500 mg, 1.70 mmol) are sequentially added to the reaction solution at 0°C. The reaction mixture is stirred at 25°C for 16 hours. LCMS (es6131-8-p1a) detection indicates complete reaction of the reactants. The reaction mixture is subjected to prep-HPLC (column: Waters Xbridge 150*25 5u; mobile phase: [water(10mM NH4HCO3)-ACN]; B%: 25%-55%, 10min), and the eluent is lyophilized to obtain a crude product . The crude product is purified by prep-TLC (dichloromethane: methanol = 15:1) to obtain **Compound 8** (59 mg, yield: 7%) as a white solid.

**¹H NMR:**(ES6131-28-P1A1, CDCl₃, 400MHz)*δ*10.20 (s, 1H), 9.72 (s, 2H), 8.72 (s, 1H), 8.60-8.58 (m, 1H), 7.95(s, 1H), 7.93-7.84 (m, 1H), 7.42 (s, 2H), 6.84 (s, 1H), 6.59-6.53 (m, 1H), 7.42 (s, 2H), 6.84 (s, 1H), 6.59-6.53 (m, 1H), 6.37-6.32 (m, 1H), 5.76-5.73 (d, *J* = 10.8 Hz, 1H), 3.92 (s, 3H), 2.91 (s, 2H), 2.75 (s, 3H), 2.31 (s, 8H).

**LCMS:** 488.3 [M+H]⁺, Rt = 0.687. **HPLC:** 97.53% purity (220 nm), Rt = 3.70.

### Example 4

### N-(5-((4-(3,3-dimethyl-2,3-dihydro-1H-pyrrolo[3,2-b]pyridin-1-yl)-1,3,5-triazin-2-yl)amino)-2 -((2-(dimethylamino)ethyl)(methyl)amino)-4-methoxyphenyl)acrylamide (Compound 11)

### Synthetic Route:

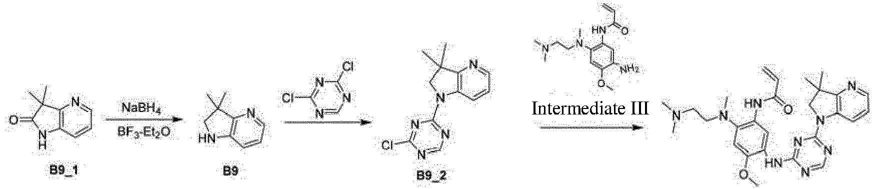

Sodium borohydride (583 mg, 15.4 mmol) is added to a solution of Compound **B9_1** (500 mg, 3.08 mmol) in tetrahydrofuran (10mL) at 0°C under the protection of nitrogen, then BF₃.Et₂O (6.82 g, 21.6 mmol, 5.93 mL, 45% purity) is added dropwise. The reaction mixture is then stirred at 20°C for 6 hours. TLC (petroleum ether: ethyl acetate = 1:1, Rf = 0.45) detection indicates complete reaction of the reactants. The reaction mixture is quenched with saturated ammonium chloride (5 mL), and extracted with ethyl acetate (10 mL). The aqueous phase is extracted with ethyl acetate (5 mL × 3). The combined organic phase is washed with saturated salt water (5 mL), then drying over sodium sulfate, and concentrating to give **Compound B9** (400 mg, yield: 88%) as a yellow solid.

**¹H NMR:**(ES6174-5-P1A 400MHz CDCl₃)δ 7.86 (dd, *J* = 1.2 Hz, 4.8 Hz, 1H), 6.98 (dd, *J* = 4.8 Hz, 8.0 Hz, 1H), 6.93 (dd, *J* = 5.2 Hz, 8.0 Hz, 1H), 3.42 (s, 2H), 1.39 (s, 6H).

Compound **B9** (200 mg, 1.40 mmol) and pyridine (213 mg, 2.70 mmol) are added to a solution of 2,4-dichloro-1,3,5-triazine (202 mg, 1.40 mmol) in tetrahydrofuran (10 mL), then stirring at 25°C for 5 hours under the protection of nitrogen. TLC (petroleum ether: ethyl acetate = 2:1) detection indicates complete reaction of the reactants. The reaction mixture is concentrated under reduced pressure to obtain a crude product. The crude product is purified by silica gel column chromatography (petroleum ether: ethyl acetate = 30: 1-25: 1) to obtain Compound **B9_2** (35 mg, yield: 10%) as a white solid.

Compound **B9_2** (35 mg, 0.13 mmol) and pyridine (21 mg, 0.27 mmol) are added to a solution of Intermediate **III** (39 mg, 0.13 mmol) in 3 mL of tetrahydrofuran, then stirring at 25°C for 5 hours under the protection of nitrogen. TLC (petroleum ether: ethyl acetate = 2:1) detection indicates complete reaction of the reactants. The reaction mixture is concentrated under reduced pressure to obtain a crude product. The crude product is purified by prep-HPLC (column: Waters Xbridge 150*25 5u; mobile phase: [water(10mM NH₄HCO₃)-ACN]; B%: 31%-51%, 8min) to give Compound **11** (9.5 mg, yield: 14%) as a yellow solid.

**¹H NMR**:(ES6176-31-P1B, CDCl₃, 400MHz)*δ*10.11-10.06 (br s, 1H), 9.72 (s, 1H), 8.60-8.58 (d, *J* = 8.4 Hz, 1H), 8.54 (s, 1H), 8.44 (s, 1H), 7.79-7.76 (br s, 1H), 7.13 (s, 1H), 6.80 (s, 1H), 6.48-6.44 (m, 1H), 6.37-6.31 (m, 1H), 5.73-5.69 (d, *J* = 11.6 Hz, 1H), 4.44-4.40 (br s, 1H), 3.88 (s, 3H), 2.90 (s, 2H), 2.72 (s, 3H), 2.25 (s, 8H), 1.50 (s, 6H), 1.43 (s, 2H).**LCMS:** 518.4 [M+H]⁺, Rt = 0.655.**HPLC:** 89.62% purity (220 nm), Rt = 3.76.

### Example 5

### N-(5-((4-(1H-indazol-1-yl)-1,3,5-triazin-2-yl)amino)-2-((2-(dimethylamino)ethyl)(methyl)ami no)-4-methoxyphenyl)acrylamide (Compound 12) (not part of the present invention)

### Synthetic Route:

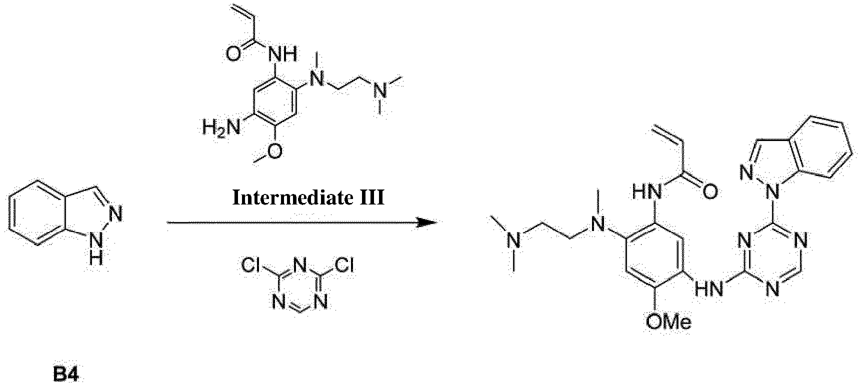

Compound **B4** (162 mg, 1.40 mmol) and sodium bicarbonate (127 mg, 1.50 mmol) are added to a solution of 2,4-dichloro-1,3,5-triazine (206 mg, 1.40 mmol) in acetonitrile (12 mL) at 0°C, then stirring at 0°C for 6 hours. Intermediate III (400 mg, 1.40 mmol) is added, and then the reaction mixture is stirred at 25°C for 20 hours. TLC (dichloromethane: methanol = 20:1, Rf = 0.40) detection indicates complete reaction of the reactants. The reaction mixture is extracted with water (12 mL) and dichloromethane (12 mL × 3). The organic phase is washed with saturated salt water, drying over sodium sulfate, filtering and concentrating to obtain a crude product. The crude product is purified by prep-HPLC, and the eluate is lyophilized to obtain **Compound 12** (110 mg, yield: 16%) as a yellow solid.

**¹H NMR:**(ES6131-28-P1A1, CDCl₃, 400MHz)*δ*10.28-10.23 (m, 2H), 9.96 (s, 1H), 8.80 (s, 1H), 8.24-8.21 (m, 1H), 7.79-7.77 (m, 1H), 7.34-7.30 (m, 1H), 7.11-7.07 (m, 1H), 6.84 (s, 1H), 6.66-6.62(d, *J* = 16.8 Hz, 1H), 6.45-6.41 (m, 1H), 5.83-5.80 (d, *J* = 9.6 Hz, 1H), 3.93 (s, 3H), 2.90-2.89 (m, 2H), 2.74 (s, 3H), 2.34-2.31 (m, 8H)_{∘}

LCMS: 488.3 [M+H]⁺, Rt = 0.688.

**HPLC:** 98.33% purity (220 nm), Rt = 4.52.

### Example 6

### N-(5-((4-(1H-pyrrolo[3,2-c]pyridin-1-yl)-1,3,5-triazin-2-yl)amino)-2-((2-(dimethylamino)ethyl )(methyl)amino)-4-methoxyphenyl)acrylamide (Compound 13) (not part of the present invention)

### Synthetic Route:

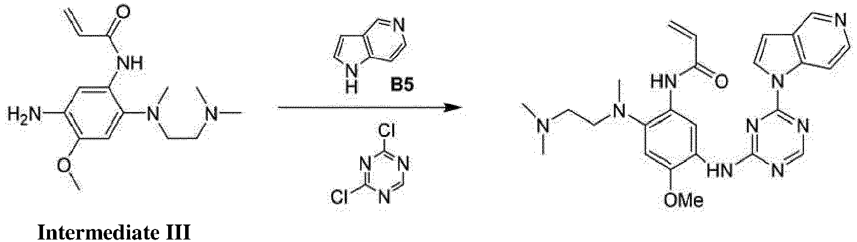

Intermediate **III** (500 mg, 1.71 mmol) and diisopropylethylamine (234 mg, 1.80 mmol) are added to a solution of 2,4-dichloro-1,3,5-triazine (257 mg, 1.70 mmol) in DMF (10 mL) at 0°C, then stirring at 0°C for 1 hour. Diisopropylethylamine (234 mg, 1.80 mmol) and Compound **B5** (202 mg, 1.71 mmol) are added sequentially. The reaction mixture is then stirred at 25°C for 15 hours. LCMS detection indicates complete reaction of the reactants. The reaction mixture is purified by prep-HPLC (column: Waters Xbridge 150*25 5u; mobile phase: [water(10mM NH₄HCO₃)-ACN]; B%: 23%-53%, 10min), the eluent is lyophilized, and then purified by prep-TLC (dichloromethane: methanol = 15: 1) to obtain **Compound 13** (60 mg, yield: 7%) as an off-white solid.

**¹H NMR:**(ES6131-13-P2A, CDCl₃, 400MHz)*δ*10.23 (s, 1H), 9.66-9.60 (m, 1H), 8.93-8.88 (m, 1H), 8.67-8.60 (m, 2H), 8.43-8.42 (m, 1H), 7.92-7.89 (m, 1H), 6.84 (s, 2H), 6.51-6.46 (d, *J* = 17.6 Hz, 1H), 6.38-6.31 (dd, *J₁* = 10.0 Hz, *J₂* = 16.8 Hz, 1H), 5.75-5.73 (d, *J* = 13.0 Hz, 1H), 3.90 (s, 3H), 2.92-2.89 (t, *J* = 6.0 Hz, 2H), 2.75 (s, 3H), 2.35-2.33 (t, *J* = 5.6 Hz, 2H), 2.30 (s, 6H).**LCMS:** 488.4 [M+H]⁺, Rt = 0.660.

**HPLC:** 97.75% purity (220 nm), Rt = 3.47.

### Example 7

### N-(2-((2-(dimethylamino)ethyl)(methyl)amino)-4-methoxy-5-((4-(3-methyl-1H-indazol-1-yl)-1,3,5-triazin-2-yl)amino)phenyl)acrylamide (Compound 14) (not part of the present invention)

### Synthetic Route:

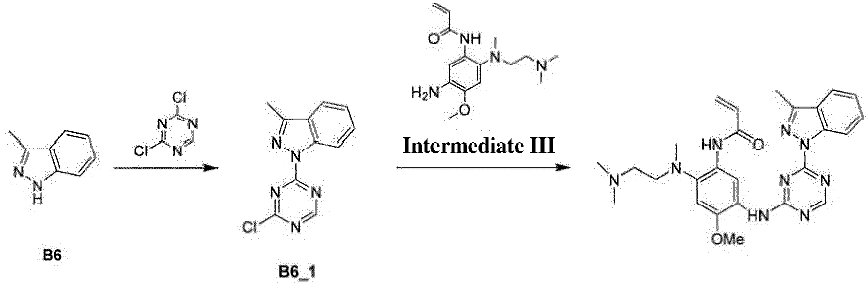

2,4-dichloro-1,3,5-triazine (340 mg, 2.30 mmol) is added to a solution of Compound **B6** (300 mg, 2.30 mmol) in acetonitrile (5 mL) at 25°C, then stirring at 50°C for 3 hours. The product is detected by LCMS. The reaction mixture is filtered and concentrated to give a crude product **B6_1** (410 mg) as a yellow solid, which is used in the next reaction without purification.

Intermediate **III** (357 mg, 1.20 mmol) is added to a solution of Compound **B6_1** (300 mg, 1.20 mmol) in DMF (10 mL) at 25°C, then stirring at 25°C for 3 hours. The product is detected by LCMS. The reaction mixture is subjected to prep-HPLC (column: Waters Xbridge 150*25 5u; mobile phase: [water(10mM NH₄HCO₃)-ACN]; B%: 25%-55%, 10min), and the eluent is lyophilized to obtain a crude product . The crude product is recrystallized in ethyl acetate (3 mL), and filtered to obtain **Compound 14** (127 mg, yield: 21%) as a yellow solid.

**¹H NMR:**(ES6131-31-plb, CDCl₃, 400MHz)*δ*9.63 (s, 1H), 9.09 (s, 1H), 8.95 (s, 1H), 8.17 (s, 1H), 7.74-7.72 (d, *J* = 8.8 Hz, 1H), 7.60-7.58 (d, *J* = 8.0 Hz, 1H), 7.33-7.30 (m, 1H), 7.06-7.03 (m, 1H), 6.94-6.90 (m, 1H), 6.72 (s, 1H), 6.51-6.47 (d, *J* = 16.8 Hz , 1H), 5.77-5.74 (d, *J* = 11.2 Hz, 1H), 3.89 (s, 3H), 3.29-3.27 (t, *J* = 5.2 Hz, 2H), 3.19 (s, 2H), 3.08 (s, 3H), 2.84 (s, 6H), 2.64 (s, 3H).**LCMS:** 502.4 [M+H]⁺, Rt = 0.692.

**HPLC:** 99.48% purity (220 nm), Rt = 3.38.

### Example 8

### N-(2-((2-(dimethylamino)ethyl)(methyl)amino)-5-((4-(5-fluoro-3-methyl-1H-indazol-1-yl)-1,3 ,5-triazin-2-yl)amino)-4-methoxyphenyl)acrylamide (Compound 15) (not part of the present invention)

### Synthetic Route:

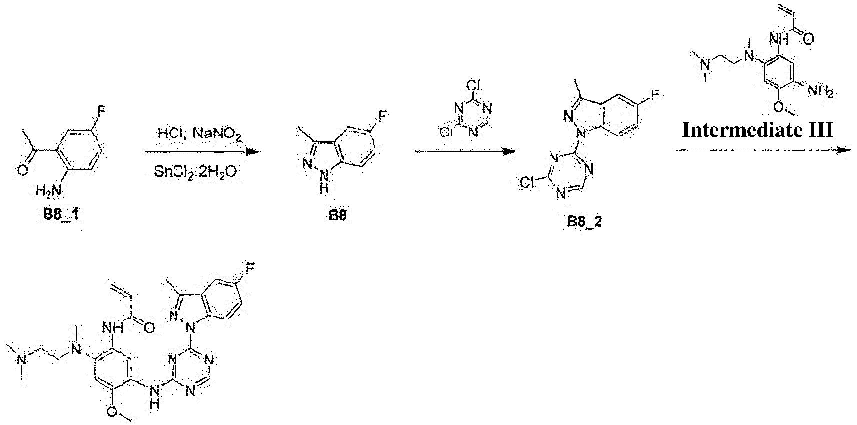

A solution of sodium nitrite (1.9 g, 27.9 mmol) in water (9.6 mL) is added dropwise to a solution of Compound **B8_1** (4.0 g, 26.1 mmol) in concentrated hydrochloric acid (20 mL) under stirring at 0°C, then stirring continuously for 1 hour. A solution of SnCl₂.2H₂O (13.2 g, 58.5 mmol) in concentrated hydrochloric acid (9.6 mL) is added dropwise to the reaction solution at 0°C, then stirring continuously for 2 hours. TLC (petroleum ether: ethyl acetate = 8:1) detection indicates complete reaction of the reactants. Saturated potassium carbonate solution is added to the reaction solution until pH = 8, then extracting with ethyl acetate (200 mL × 4). The organic phase is washed with saturated salt water (150 mL), then drying over sodium sulfate, filtering and concentrating under reduced pressure to obtain yellow Compound **B8** (3.9 g, yield: 51%).

**¹H NMR**:(ES6176-15-P1A, CDCl₃, 400MHz)*δ*10.01 (br s, 1H), 7.39-7.36 (m, 1H), 7.32-7.29 (dd, *J*₁= 2.0 Hz, J₂= 8.8 Hz, 1H), 7.19-7.14 (td, *J₁*= 2.4 Hz, *J*₂= 8.8 Hz, 1H), 2.57 (s, 3H).

Compound **B8** (501 mg, 3.33 mmol) and sodium bicarbonate (308 mg, 3.66 mmol) are added to a solution of compound 2,4-dichloro-1,3,5-triazine (500 mg, 3.33 mmol) in acetonitrile (10 mL) at 0°C. The reaction mixture is then stirred at 25°C for 16 hours under the protection of nitrogen. TLC (dichloromethane: petroleum ether = 5:1) detection indicates complete reaction of the reactants. The reaction mixture is filtered, and the filtrate is concentrated under reduced pressure to obtain a crude product. The crude product is purified by silica gel column chromatography (dichloromethane: petroleum ether = 1:1 to 4:1) to obtain yellow Compound **B8_2** (400 mg, yield: 46%).

**¹H NMR:**(ES6176-21-P1A2, CDCl₃, 400MHz)*δ*9.12 (s, 1H), 7.72-7.69 (m, 1H), 7.18-7.10 (m, 2H), 3.02 (s, 3H).

Compound **B8_2** (200 mg, 0.76 mmol) is added to a solution of Intermediate **III** (222 mg, 0.76 mmol) in tetrahydrofuran (10 mL) at 25°C, and then pyridine (120 mg, 1.50 mmol) is added to the reaction solution. The reaction mixture is stirred at 25°C for 6 hours under the protection of nitrogen. TLC (petroleum ether: ethyl acetate = 4:1) detection indicates complete reaction of the reactants. The reaction mixture is concentrated under reduced pressure to obtain a crude product. The crude product is purified by prep-HPLC (column: Gemini 150*25 5u; mobile phase: [water(10 mM NH₄HCO₃)-ACN]; B%: 35%-60%, 12.5min) to give **Compound 15** (46 mg, yield: 12%) as a white solid.

**¹H NMR:**(ES6176-23-P1A,CDCl₃, 400MHz)*δ*10.19 (s, 1H), 9.60 (s, 1H), 8.14 (s, 1H), 7.72 (br s, 1H), 7.15-7.11 (m, 2H), 6.82 (s, 1H), 6.49-6.45 (m, 1H), 6.35-6.29 (m, 1H), 5.73-5.70 (d, *J =* 9.6 Hz, 1H), 3.88 (s, 3H), 3.02 (s, 2H), 2.84 (s, 2H), 2.73 (s, 3H), 2.66 (s, 1H), 2.33 (s, 3H), 2.29 (s, 6H).

**LCMS:** 520.4 [M+H]⁺, Rt = 0.695. **HPLC**: 98.4% purity (220 nm), Rt = 3.75.

### Example 9

### N-(2-((2-(dimethylamino)ethyl)(methyl)amino)-4-methoxy-5-((4-(3-methyl-1H-pyrrolo[3,2-c) pyridin-1-yl)-1,3,5-triazin-2-yl)amino)phenyl)acrylamide (Compound 18)

### Synthetic Route:

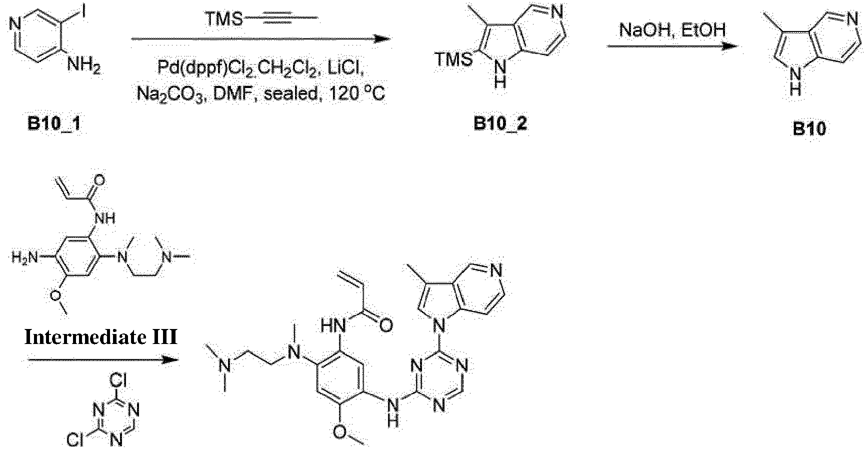

Trimethyl(prop-1-ynyl)silane (1.5 g, 13.6 mmol), lithium chloride (192.9 mg, 4.50 mmol), sodium carbonate (964.5 mg, 9.10 mmol), and Pd (dppf)Cl₂.CH₂Cl₂ (186 mg, 0.23 mmol) are added to a solution of Compound **B10_1** (1.0 g, 4.50 mmol) in DMF (15 mL), and then the reaction mixture is stirred at 25°C for 16 hours under the protection of nitrogen. TLC (petroleum ether: ethyl acetate = 1:1) detection indicates complete reaction of the reactants. Water (80 mL) is added to the reaction solution, then extracting with ethyl acetate (100 mL × 3). The organic phase is washed with saturated salt water, then drying over sodium sulfate, filtering and concentrating under reduced pressure to obtain a crude product. The crude product is purified by silica gel column chromatography (petroleum ether: ethyl acetate = 20:1 to 1:1) to obtain a brown solid **B10_2** (780 mg, yield: 84%).

**¹H NMR:**(ES6176-36-P1A, CDCl₃, 400MHz)*δ*8.89 (s, 1H), 8.70 (br s, 1H), 8.27-8.26 (d, *J* = 5.6 Hz, 1H), 7.24-7.22 (d, *J* = 6.0 Hz, 1H), 2.47 (s, 3H), 0.40 (s, 9H).

Sodium hydroxide (4.7 g, 117 mmol) is added to a solution of Compound **B10_2** (780 mg, 3.80 mmol) in ethanol (35 mL), and then the reaction mixture is stirred at 80°C for 16 hours. TLC (dichloromethane: methanol = 20:1) detection indicates complete reaction of the reactants. The reaction solution is cooled to 25°C, then pouring into water (50 mL), and extracting with ethyl acetate (60 mL × 3). The organic phase is washed with saturated salt solution (50 mL), then drying over sodium sulfate, filtering and concentrating under reduced pressure to obtain a crude product. The crude product is purified by silica gel column chromatography (dichloromethane: methanol = 100: 1-20: 1) to obtain a brown solid **B10** (400 mg, yield: 79%).

**¹H NMR:**(ES6176-39-P1A, CDCl₃, 400MHz)*δ*9.49 (br s, 1H), 8.70 (s, 1H), 8.09-8.07 (d, *J* = 5.6 Hz, 1H), 7.07-7.06 (d, *J* = 6.0 Hz, 1H), 6.83 (s, 1H), 2.19 (s, 3H).

Compound 2,4-dichloro-1,3,5-triazine (340 mg, 2.30 mmol) is added to a solution of Intermediate **III** (664 mg, 2.3 mmol) in DMF (10 mL) at 0°C, and then the reaction mixture is stirred at 0°C for 1 hour under the protection of nitrogen. Compound **B10** (300 mg, 2.30 mmol) is added to the reaction solution, and then the reaction mixture is stirred at 0°C for 2 hours under the protection of nitrogen. The target product is detected by LCMS. The reaction mixture is concentrated under reduced pressure to obtain a crude product. The crude product is purified by prep-HPLC (column: Waters Xbridge 150*25 5u; mobile phase: [water(10mM NH₄HCO₃)-ACN]; B%: 18%-48%, 8min), and then purified by prep-HPLC (column: Waters Xbridge 150*25 5u; mobilephase: [water(10mM NH₄HCO₃)-ACN]; B%: 32%-62%, 10min) to obtain **Compound 18** (58mg, yield: 5%) as a white solid.

**¹H NMR:**(ES6176-42-PlA,CDCl₃, 400MHz)*δ*10.19 (s, 1H), 9.76-9.74 (br s, 1H), 8.85 (s, 2H), 8.65-8.59 (m, 2H), 8.47 (s, 1H), 7.97-7.96 (br, 1H), 6.83 (s, 1H), 6.54-6.39 (m, 2H), 5.76-5.74 (d, *J* = 10.4 Hz, 1H), 3.91 (s, 3H), 2.94-2.91 (m, 2H), 2.74 (s, 3H), 2.49 (s, 3H), 2.38 (s, 2H), 2.33 (s, 6H).**LCMS:** 502.3 [M+H]⁺, Rt = 0.632.

**HPLC:** 100.0% purity (220 nm), Rt = 3.85.

### Example 10

### N-(5-((4-(1H-benzo[d] [1,2,3]triazol-1-yl)-1,3,5-triazin-2-yl)amino)-2-((2-(dimethylamino)ethy 1)(methyl)amino)-4-methoxyphenyl)acrylamide (Compound 19) (not part of the present invention)

### Synthetic Route:

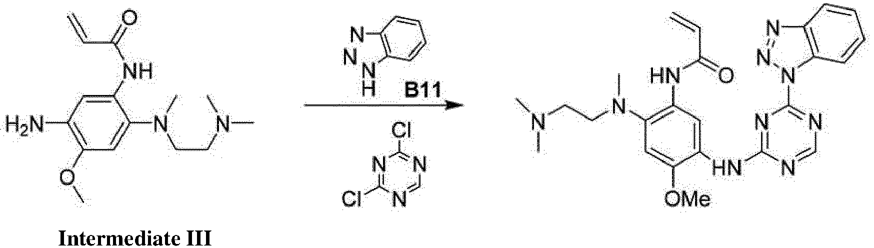

Intermediate **III** (204 mg, 1.70 mmol) and diisopropylethylamine (234 mg, 1.80 mmol) are added to a solution of compound 2,4-dichloro-1,3,5-triazine (257 mg, 1.70 mmol) in DMF (10 mL) at 0°C, and then the reaction mixture is stirred at 0°C for 1 hour. Diisopropylethylamine (234 mg, 1.80 mmol) and Compound **B11** (204 mg, 1.70 mmol) are sequentially added to the reaction solution. The reaction mixture is then stirred at 25°C for 15 hours. LCMS detection indicates complete reaction of the reactants. The reaction solution is directly purified by prep-HPLC (column: Waters Xbridge 150*25 5u; mobile phase: [water(10mM NH₄HCO₃)-ACN]; B%: 25%-55%, 10min), and lyophilized to obtain a crude product (190mg). The crude product is further purified by prep-TLC (dichloromethane: methanol = 20:1) to obtain **Compound 19** (79 mg, yield: 13%) as a yellow solid.

**¹H NMR:**(ES6131-11-P1A, CDCl₃, 400MHz)*δ*10.22 (s, 1H), 9.03 (s, 1H), 9.02 (s, 1H), 8.66-8.64 (d, *J* = 7.6 Hz, 1H), 8.19-8.17 (d, *J* = 8.8 Hz, 2H), 7.69 (s, 1H), 7.51 (s, 1H), 6.84 (s, 1H), 6.55-6.33 (m, 2H), 5.73-7.70 (m, 1H), 3.91 (s, 3H), 6.79 (s, 1H), 2.89 (s, 2H), 2.74 (s, 3H), 2.29 (s, 8H).

**LCMS:** 489.2 [M+H]⁺, Rt = 0.673. **HPLC:** 95.63% purity (220 nm), Rt = 3.27.

### Example 11

### N-(2-((2-(dimethylamino)ethyl)(methyl)amino)-5-((4-(6-fluoro-3-methyl-1H-indazol-1-yl)-1,3 ,5-triazin-2-yl)amino)-4-methoxyphenyl)acrylamide (Compound 20)

### Synthetic Route:

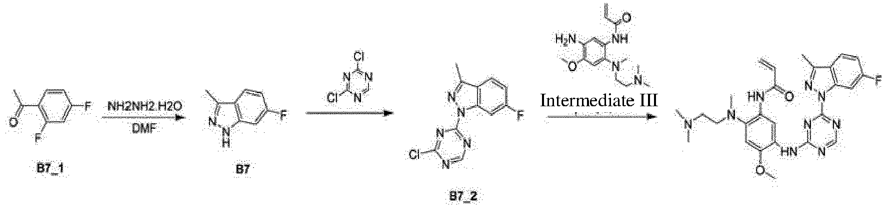

Compound **B7_1** (10.0 g, 64.1 mmol) is added to a solution of hydrazine hydrate (6.4 g, 128 mmol) in DMF (50 mL) at 25°C. The reaction mixture is then stirred at 120°C for 18 hours under the protection of nitrogen. TLC (petroleum ether: ethyl acetate = 5:1) detection indicates complete reaction of the reactants. The reaction solution is cooled to room temperature, then diluting with water (200 mL), and extracting with ethyl acetate (150 mL × 3). The organic phase is washed with saturated salt water (200 mL), then drying over sodium sulfate, filtering and concentrating under reduced pressure to obtain a crude product. The crude product is purified by silica gel column chromatography (petroleum ether: ethyl acetate = 5:1 to 1:1) to obtain **Compound B7** (3.0 g, yield: 31%) as a white solid.

**¹H NMR:**(ES6176-3-PlAl,CDCl₃, 400MHz)δ10.35 (br s, 3H), 7.64-7.60 (m, 1H), 7.09-7.06 (dd, *J₁*= 2.0 Hz, *J₂*= 9.2 Hz, 1H), 6.95-6.92 (td, *J₁*= 8.8 Hz, *J₂*= 11.2 Hz, 1H), 2.59 (s, 3H).

Compound **B7** (501 mg, 3.33 mmol) and sodium bicarbonate (308 mg, 3.66 mmol) are added to a solution of compound 2,4-dichloro-1,3,5-triazine (500 mg, 3.33 mmol) in acetonitrile (10 mL) at 0°C, and then the reaction mixture is stirred at 25°C for 16 hours under the protection of nitrogen. TLC (dichloromethane: petroleum ether = 4:1) detection indicates complete reaction of the reactants. The reaction solution is filtered, and the filtrate is concentrated under reduced pressure to obtain a crude product. The crude product is purified by silica gel column chromatography (dichloromethane: petroleum ether = 1:1 to 3:1) to obtain **Compound B7_2** (410 mg, yield: 47%) as a yellow solid.

**¹H NMR:**(ES6176-3-P1A1,CDCl₃, 400MHz)*δ*8.97 (s, 1H), 8.42-8.39 (dd, *J₁*= 1.6 Hz, *J₂*= 9.6 Hz, 1H), 7.70-7.67 (m, 1H), 7.21-7.17 (td, *J₁*= 2.0 Hz, *J₂*= 4.4 Hz, 1H), 2.68 (s, 3H).

Compound **B7_2** (200 mg, 0.76 mmol) and pyridine (120 mg, 1.5 mmol) are added to a solution of Intermediate **III** (222 mg, 0.76 mmol) in tetrahydrofuran (10 mL) at 25°C. The reaction mixture is then stirred at 25°C for 6 hours under the protection of nitrogen. TLC (petroleum ether: ethyl acetate = 4: 1) detection indicates complete reaction of the reactants. The reaction solution is concentrated under reduced pressure to obtain a crude product. The crude product is purified by prep-HPLC (column: Gemini 150*25 5u; mobile phase: [water(10 mM NH₄HCO₃)-ACN]; B%: 35%-60%, 12.5min) to obtain **Compound 20** (76mg, yield: 19%) as a white solid.

**¹H NMR:**(ES6176-24-P1A1,CDCl₃, 400MHz)*δ*10.20-10.13 (m, 1H), 9.59 (s, 1H), 8.90 (s, 1H), 8.15 (s, 1H), 7.63-7.58 (br s, 1H), 7.31 (s, 1H), 6.88-6.82 (m, 2H), 6.48-6.44 (m, 1H), 6.35-6.29 (m, 1H), 5.73-5.70 (d, *J* = 10.8 Hz, 1H), 3.88 (s, 3H), 3.06 (s, 2H), 2.90 (s, 2H), 2.73 (s, 3H), 2.66 (s, 1H), 2.34 (s, 2H), 2.29 (s, 6H)_{∘}

**LCMS**: 520.4 [M+H]⁺, Rt = 0.689. **HPLC**: 97.8% purity (220 nm), Rt = 3.67.

### Example 12

N-(2-((2-(dimethylamino)ethyl)(methyl)amino)-4-methoxy-5-((4-(8-methylimidazolo[1,2-a]py ridin-3-yl)-1,3,5-triazin-2-yl)amino)phenyl)acrylamide (Compound 26),
N-(2-((2-(dimethylamino)ethyl)(methyl)amino)-5-((4-(8-fluoroimidazolo[1,2-a]pyridin-3-yl)-1 ,3,5-triazin-2-yl)amino)-4-methoxyphenyl)acrylamide (Compound 27),
N-(2-((2-(dimethylamino)ethyl)(methyl)amino)-4-methoxy-5-((4-(8-(trifluoromethyl)imidazol o[1,2-a]pyridin-3-yl)-1,3,5-triazin-2-yl)amino)phenyl)acrylamide (Compound 28)

The synthetic route is as follows:

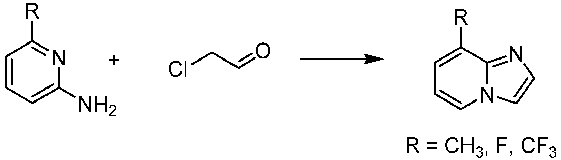

Aminopyridine (50 mmol, 1.0 eq.), chloroacetaldehyde (55 mmol, 1.1 eq.) and sodium bicarbonate (60 mmol, 1.2 mmol) are added to a round bottom flask, then adding 60 mL of ethanol and 15 mL of water to react at 80°C for 5 h (overnight at R = CF₃), and completion of the reaction is detected by TLC. 100 mL of saturated salt water is added, then extracting with dichloromethane (100 mL×3), the organic phase is dried and concentrated, and then purified by column chromatography (the eluent is: EA:PE=1:3), yield: 95%.

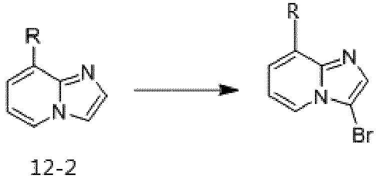

Compound 12-2 (10 mmol, 1.0 eq.) is dissolved in 20 mL of dichloromethane, and then NBS (10.5 mmol, 1.05 mmol) is added in batches; the reaction is completed by stirring at room temperature for 10 min, and quenched with saturated salt water. The resulting product is purified by column chromatography (the eluent is: EA:PE=1:4), yield: 95%.

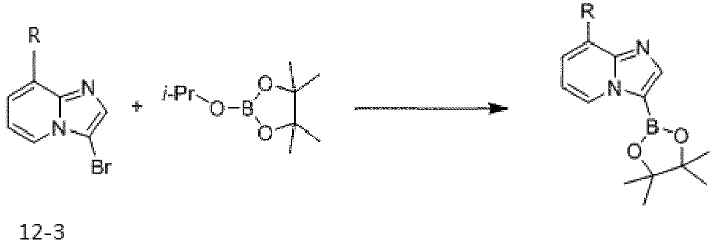

Compound **12-3** (10 mmol, 1.0 eq.) and isopropyl pinacolyl borate (10 mmol, 1.0 eq.) are dissolved in 20 mL of tetrahydrofuran under the protection of argon. Isopropylmagnesium chloride tetrahydrofuran solution (2.0 M, 5.5 mL, 1.1 eq.) is added dropwise under ice bath to react for 3 h. The reaction is quenched with saturated salt water, then extracting with ethyl acetate (50 mL×3). The organic phase is dried and concentrated, and then purified by column chromatography (the eluent is: EA: MeOH = 12:1), yield: 25%.

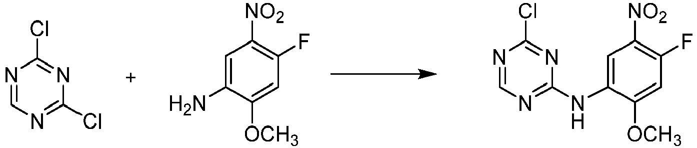

2,4-dichloro-1,3,5-triazine (10 mmol, 1.0 eq.) is dissolved in 20 mL of acetone, then dropwise adding 4-fluoro-2-methoxy-5-nitroaniline (9.5 mmol, 0.95 eq.) in acetone (10 mL) under ice bath to react for 3 h. The resulting product is purified by column chromatography (the eluent is: EA: PE=1:4), yield: 50%.

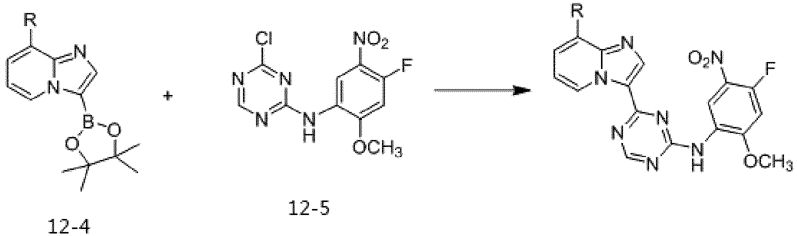

Compound **12-4** (1.0 mmol, 1.0 eq.) and Compound **12-5** (1.0 mmol, 1.0 eq.) are dissolved in 2.0 mL of DMSO under the protection of argon, then adding tetratriphenylphosphine palladium (0.15 mmol, 0.15 eq.) and sodium carbonate solution (1.0 M, 2.0 eq.), and placing in a microwave reactor to react at 120°C for 1 h, filtering and concentrating, and the resulting solution is directly used in the next reaction.

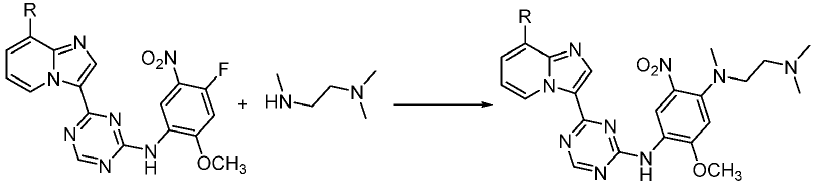

N,N,N'-trimethylethylenediamine (1.0 mmol, 1.0 eq.) and N,N-diisopropylethylamine (2.0 mmol, 2.0 eq.) are added to the resulting solution in the previous step, then placing in a microwave reactor to react at 140°C for 1 h; and then 50 mL of ethyl acetate is added, washing with a large amount of saturated salt water; the organic phase is retained, dried and concentrated, and the resulting concentrate is used directly in the next reaction.

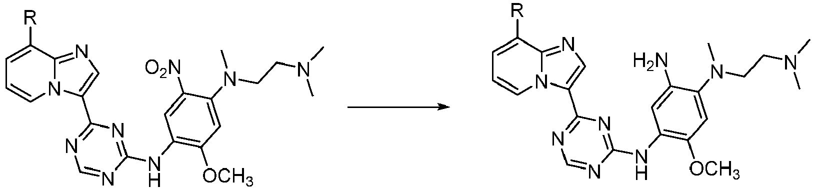

Ethanol (5 mL) and water (1 mL) are added to the resulting concentrate in the previous step, then adding iron powder (10.0 mmol, 10.0 eq.), ammonium chloride (10.0 mmol, 10.0 eq.), and stirring at room temperature overnight; adjusting the pH to 8-9 with saturated sodium bicarbonate solution, then extracting with ethyl acetate; the organic phase is retained, dried and concentrated, and the resulting concentrate is used directly in the next reaction.

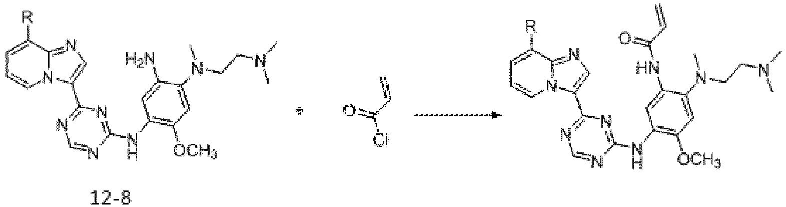

Compound **12-8** (1.0 mmol, 1.0 eq.) is dissolved in dichloromethane under the protection of argon, then adding acryloyl chloride (1.0 mmol, 1.0 eq.) and N,N-diisopropylethylamine (1.0 mmol, 1.0 eq.), and stirring at room temperature for 20 min; the reaction is quenched with saturated salt water, then extracting with dichloromethane (20 mL×3). The organic phase is retained, dried and concentrated, then purifying with preparative high-performance liquid chromatography.

### Example 13

### N-(2-((2-(dimethylamino)ethyl)(methyl)amino)-5-((4-(3-fluoro-1H-indazol-1-yl)-1,3,5-triazin-2-yl)amino)-4-methoxyphenyl)acrylamide (Compound 29)

The synthesis method is as follows:

Compound **13-1** (200 mg, 1.47 mmol) and Compound **13-2** (420 mg, 1.40 mmol) are dissolved in CH₃CN (20 ml), then adding K₂CO₃ (386.7 mg, 2.80 mmol) and stirring at room temperature overnight. The reaction progress is detected by LC-MS. After the reaction is completed, water is added for quenching, then extracting with ethyl acetate (50 ml × 3), drying over anhydrous sodium sulfate; and a crude product Compound **13-3** is obtained by rotary drying.

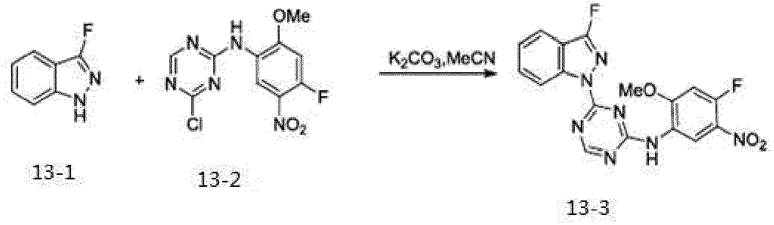

Compound **13-3** obtained in the previous step and Compound **13-4** (123.5 mg, 1.2 mmol) are dissolved in DMA (10 ml), then adding DIPEA (260.4 mg, 2.0 mmol) to react under microwave condition at 80°C for 1 hour. LC-MS is used to detect the progress of the reaction. After the reaction is completed, water is added for quenching, then extracting with ethyl acetate (3 ml × 3), and drying over anhydrous sodium sulfate; and a crude product Compound **13-5** is obtained by rotary drying.

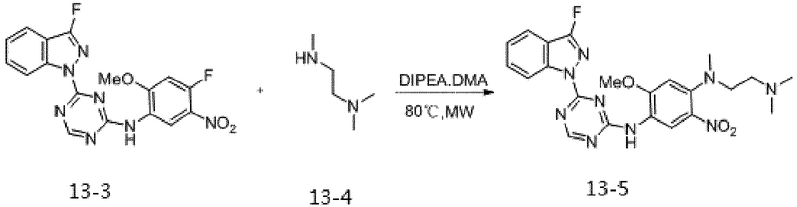

Compound **13-5** obtained in the previous step is dissolved in CH₃OH (20 ml), then adding Pd/C (50 mg), and the mixture is reduced overnight at one standard atmospheric pressure and H₂ atmosphere. The progress of the reaction is detected by LC-MS. After the reaction is completed, Pd/C is filtered off with suction, then washing with ethyl acetate (5 ml × 3), and a crude product Compound **13-6** is obtained by rotary drying.

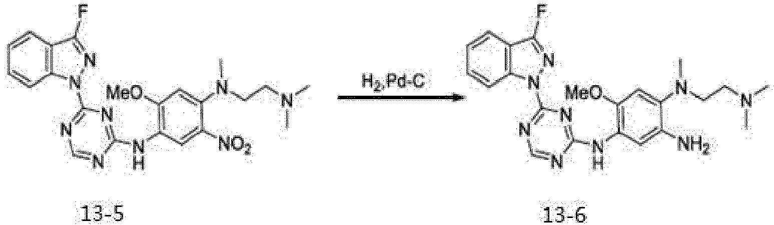

The crude product **13-6** obtained in the previous step and DIPEA (219.7 mg, 1.7 mmol) are dissolved in DCM (20 ml), then adding Compound **13-7** (67.7 mg, 0.75 mmol), reacting at room temperature for 30 min. The progress of the reaction is detected by LC-MS. After the reaction is completed, it is quenched with saturated sodium bicarbonate solution, then extracting with ethyl acetate (30 ml × 3). A crude product **13-8** is obtained by rotary drying, then separating it with preparative liquid chromatography to obtain Compound **13-8** (10 mg) as a white solid. 1H NMR (500 MHz, DMSO-d6) 6 9.84 (br, 1H), 9.58 (br, 1H), 9.38 (br, 1H), 8.66 (br, 1H), 8.02 (s, 1H), 7.88 (br, 1H), 7.46 (m, 2H), 7.08 (m, 1H), 6.66 (m, 1H), 6.28 (d, *J* = 17 Hz, 1H), 5.78 (d, *J* = 12 Hz, 1H), 3.82 (s, 3H), 3.33 (m, 7 H), 2.83 (s, 3H), 2.82 (s, 3H), 2.64 (m, 4H). ¹⁹F NMR (376MHz, DMSO-*d₆*)*δ*-73.7. HRMS (ESI) calcd.for [C₂₅H₂₈FN₉O₂H]⁺(M+H)⁺: m/z 506.24228.

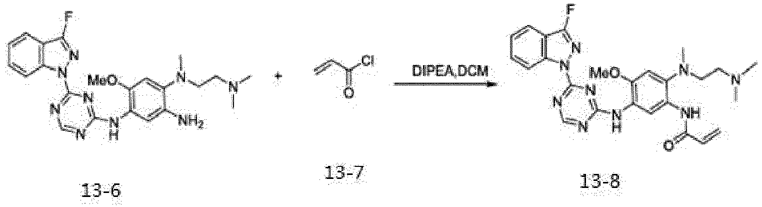

Table 1 below is the LC/MS data of each example.

**Table 1:**

| Example No. | Compound No. | [M+H]⁺ |
|---|---|---|
| 1 | Compound 4 (not part of the present invention) | 501.4 |
| 2 | Compound 6 | 488.1 |
| 3 | Compound 8 (not part of the present invention) | 488.3 |
| 4 | Compound 11 | 518.4 |
| 5 | Compound 12 (not part of the present invention) | 488.3 |
| 6 | Compound 13 (not part of the present invention) | 488.4 |
| 7 | Compound 14 (not part of the present invention) | 502.4 |
| 8 | Compound 15 (not part of the present invention) | 520.4 |
| 9 | Compound 18 | 502.3 |
| 10 | Compound 19 (not part of the present invention) | 489.2 |
| 11 | Compound 20 | 520.4 |
| 12 | Compound 26 | 502.3 |
| 12 | Compound 27 | 506.2 |
| 12 | Compound 28 | 556.2 |
| 13 | Compound 29 | 506.2 |

### Test 1: Phosphorylation Test of L858R/T790M EGFR (double mutant) Cells

The human lung cell line NCI-H1975 (L858R/T790M double mutant EGFR) is obtained from the American Type Culture Collection. NCI-H1975 cells are cultured in RPMI1640 medium containing 10% fetal bovine serum and 2 mM glutamine. Cells are grown in a humidified incubator with 5% CO₂ at 37°C.

The detection of phosphorylated p-EGFR of endogenous cells in cell lysate is performed according to the protocol described in the DuoSet IC Human Phospho-EGFR ELISA (R&D Systems catalog number: # DYC1095) of R&D Systems Company. 90 µl of cells (8000 cells/well) are cultured in growth medium in a Corning 96-well plate with black transparent bottom, after culturing in a humidified incubator with 5% CO₂ at 37°C overnight, the cell culture medium is replaced with RPMI1640 medium containing 1% fetal bovine serum and 2 mM glutamine to culture continuously. The compound serially diluted in 100% DMSO is added to the cells by pipette, and after gently mixing the medium, the cells are incubated for another 2 hours. 100 µl of 1X lysis buffer is added to each well. Greiner high-binding 96-well plates are coated with capture antibody, and then blocked with 3% BSA. After removing the blocking solution, 100 µl of lysate is transferred to a Greiner high-binding 96-well plate, then incubating for 2 hours. After gently mixing and washing the culture plate with PBS, 100 µl of detection antibody is added, then incubating for 2 hours. After gently mixing and washing the culture plate with PBS, 100 µl of TMB substrate (Cell Signaling Technology catalog number: 7004) is added, then incubating for 1 hour. 100 µl of stop solution is added to the culture plate, and the absorbance of each well at 450 nm wavelength is measured. Finally, the data is input into a suitable software package (such as Prism) for curve fitting analysis. Based on this data, the concentration of the compound required to obtain 50% inhibition effect is obtained by calculation so as to determine the IC₅₀ value.

Table 2 below shows the IC₅₀ values (µM) of the representative compounds of the present invention (i.e., Compound 6 of Example 2; Compound 11 of Example 4; Compound 26, Compound 27, and Compound 28 of Example 12; and Compound 29 of Example 13) in the above biological experiments, wherein Osimertinib (AZD9291) as a control compound is synthesized according to the method described in the following literature:
Discovery of a Potent and Selective EGFR Inhibitor (AZD9291) of Both Sensitizing and T790M Resistance Mutations That Spares the Wild Type Form of the Receptor, J. Med. Chem., 2014, 57 (20), pp 8249-8267.

**Table 2:**

| Example No. | Compound No. | Test 1 |
|---|---|---|
| 2 | Compound 6 | 0.0151 |
| 4 | Compound 11 | 0.0111 |
| 12 | Compound 26 | 0.0121 |
| 12 | Compound 27 | 0.0118 |
| 12 | Compound 28 | 0.0114 |
| 13 | Compound 29 | 0.0106 |
| Osimertinib (AZD9291) | | 0.0459 |

As shown in Table 2 above, the IC₅₀ values of Compound 6, Compound 11, Compound 26, Compound 27, Compound 28, and Compound 29 according to the present invention in the phosphorylation test of L858R/T790M EGFR (double mutant) cells are 0.0151, 0.0111, 0.0121, 0.0118, 0.0114 and 0.0106, respectively; as compared with the above compounds, the IC₅₀ value of Osimertinib (AZD9291) in the phosphorylation test of L858R/T790M EGFR (double mutant) cells is 0.0459, which is equivalent to three times or more of the IC₅₀ value when using Compound 6 or Compound 11 of the present invention.

### Test 2: Proliferation Test of Wild-type EGFR Cells

The human lung cell line A431 (wild-type EGFR) is obtained from the American Type Culture Collection. NCI-H838 cells are cultured in RPMI1640 medium containing 10% fetal bovine serum and 2 mM glutamine. Cells are grown in a humidified incubator with 5% CO₂ at 37°C.

The number of viable cells in the culture is determined according to the protocol described in Cell Titer-Glo Luminescent cell Viability Assay (Promega catalog number: # G7570) of Promega company. 90 µl of cells (8000 cells/well) are cultured in growth medium in a Corning 96-well plate with black transparent bottom, culturing in a humidified incubator with 5% CO₂ at 37°C overnight. The compound serially diluted in 100% DMSO is added to the cells by pipette, culturing the cells for another 72 hours. 100 µl of the mixed Cell Titer-Glo reagent is added to the cells in the 96-well culture plate to lyse the cells, mixing gently. Subsequently, the autofluorescence is detected on the Envision microplate detector to obtain data of the compound. Finally, the data is input into a suitable software package (such as Prism) for curve fitting analysis. Based on this data, the concentration of the compound required to obtain 50% inhibition effect is obtained by calculation so as to determine the IC₅₀ value.

### Test 3: Proliferation Test of L858R/T790M EGFR (double mutant) cells

The human lung cell line NCI-H1975 (L858R/T790M double mutant EGFR) is obtained from the American Type Culture Collection. NCI-H1975 cells are cultured in RPMI1640 medium containing 10% fetal bovine serum and 2 mM glutamine. Cells are grown in a humidified incubator with 5% CO₂ at 37°C.

The number of viable cells in the culture is determined according to the protocol described in Cell Titer-Glo Luminescent cell Viability Assay (Promega catalog number: # G7570) of Promega company. 90 µl of cells (8000 cells/well) are cultured in growth medium in a Corning 96-well plate with black transparent bottom, culturing in a humidified incubator with 5% CO₂ at 37°C overnight. The compound serially diluted in 100% DMSO is added to the cells by pipette, culturing the cells for another 72 hours. 100 µl of the mixed Cell Titer-Glo reagent is added to the cells in the 96-well culture plate to lyse the cells, mixing gently. Subsequently, the autofluorescence is detected on the Envision microplate detector to obtain data of the compound. Finally, the data is input into a suitable software package (such as Prism) for curve fitting analysis. Based on this data, the concentration of the compound required to obtain 50% inhibition effect is obtained by calculation so as to determine the IC₅₀ value.

Table 3 below shows the IC₅₀ values (µM) of the representative compounds of the present invention (i.e., Compound 6, Compound 26, Compound 27, Compound 28, and Compound 29) in the above biological experiments, wherein Osimertinib (AZD9291) as a control compound is synthesized according to the method described in the following literature:
Discovery of a Potent and Selective EGFR Inhibitor (AZD9291) of Both Sensitizing and T790M Resistance Mutations That Spares the Wild Type Form of the Receptor, J. Med. Chem., 2014, 57 (20), pp 8249-8267.

**Table 3:**

| Example No. | Compound No. | Test 2 | Test 3 | Ratio of IC₅₀ value of Test 2 to that of Test 3 |
|---|---|---|---|---|
| 2 | Compound 6 | 0.579 | 0.003 | 193 |
| 12 | Compound 26 | 0.718 | 0.004 | 179.5 |
| 12 | Compound 27 | 0.883 | 0.005 | 176.6 |
| 12 | Compound 28 | 0.758 | 0.004 | 189.5 |
| 13 | Compound 29 | 0.941 | 0.004 | 235.3 |
| Osimertinib (AZD9291) | - | 0.344 | 0.023 | 14.9 |

As shown in Table 3, the compound of the present invention has a relatively smaller IC₅₀ value than that of Osimertinib (AZD9291) in the proliferation test of L858R/T790MEGFR (double mutant) cells. The IC₅₀ value obtained with Osimertinib (AZD9291) is about four times greater than the IC₅₀ value obtained with the compound of the present invention. This shows that the compound of the present invention may inhibit proliferation of L858R/T790M EGFR (double mutant) cells better than Osimertinib (AZD9291). In addition, as shown in Table 3, the right-most column of the table shows the ratio of the IC₅₀ value of Test 2 to that of Test 3, which characterizes the selectivity of the compound on inhibitory effect of L858R/T790M EGFR (double mutant) cell proliferation and wild-type EGFR cell proliferation. A larger value indicates that the compound has better selectivity for inhibition of L858R/T790M EGFR (double mutant) cell proliferation among wild-type EGFR cells. As shown in Table 3, the selectivity of the inhibitory effect of the compound according to the present invention on L858R/T790M EGFR (double mutant) cell proliferation is much better than that of Osimertinib (AZD9291).

### Test 4: Proliferation Test of BalF3-CAT-EGFR cells

BalF3-CAT-EGFR cell viability detection is based on the detection technology developed by CellTiter-Glo^{®}. BalF3 cells are transformed by overexpressing the intracellular kinase region of wild-type EGFR to construct a CAT-EGFR tool cell strain. This cell strain lives depending on the kinase activity of EGFR, if the wild-type EGFR is inhibited by a small molecule compound, the cell strain will die. Therefore, the specificity and activity of EGFR small molecules on wild-type EGFR may be evaluated by observing the effect of EGFR small molecules on cell viability.

Ba/F3-CAT-EGFR (wild-type EGFR) cells are cultured in RPMI1640 medium containing 10% fetal bovine serum and 2 mM glutamine. Cells are grown in a humidified incubator with 5% CO₂ at 37°C.

The number of viable cells in the culture is determined according to the protocol described in Cell Titer-Glo Luminescent cell Viability Assay (Promega catalog number: # G7570) of Promega company. 100 µl of cells (3000 cells/well) are cultured in growth medium in a Corning 96-well plate with black transparent bottom, culturing in a humidified incubator with 5% CO₂ at 37°C overnight. The compound serially diluted in 100% DMSO is added to the cells by pipette, culturing the cells for another 72 hours. 100 µl of the mixed Cell Titer-Glo reagent is added to the cells in the 96-well culture plate to lyse the cells, mixing gently. Subsequently, the autofluorescence is detected on the Envision microplate detector to obtain data of the compound. Finally, the data is input into a suitable software package (such as Prism) for curve fitting analysis. Based on this data, the concentration of the compound required to obtain 50% inhibition effect is obtained by calculation so as to determine the IC₅₀ value.

### Test 5: Proliferation Test of Ba/F3-DTM-EGFR cells

Ba/F3-DTM-EGFR cell viability detection is based on the detection technology developed by CellTiter-Glo^{®}. BalF3 cells are transformed by overexpressing the wild-type T790M mutation and Exon19-deficient EGFR to construct a DTM-EGFR tool cell strain. This cell strain lives depending on the kinase activity of EGFR, if the mutant EGFR is inhibited by a small molecule compound, the cell strain will die. Therefore, the specificity and activity of EGFR small molecules on mutant EGFR may be evaluated by observing the effect of EGFR small molecules on cell viability.

BalF3-DTM-EGFR (mutant EGFR) cells are cultured in RPMI1640 medium containing 10% fetal bovine serum and 2 mM glutamine. Cells are grown in a humidified incubator with 5% CO₂ at 37°C.

The number of viable cells in the culture is determined according to the protocol described in Cell Titer-Glo Luminescent cell Viability Assay (Promega catalog number: # G7570) of Promega company. 100 µl of cells (3000 cells/well) are cultured in growth medium in a Corning 96-well plate with black transparent bottom, culturing in a humidified incubator with 5% CO₂ at 37°C overnight. The compound serially diluted in 100% DMSO is added to the cells by pipette, culturing the cells for another 72 hours. 100 µl of the mixed Cell Titer-Glo reagent is added to the cells in the 96-well culture plate to lyse the cells, mixing gently. Subsequently, the autofluorescence is detected on the Envision microplate detector to obtain data of the compound. Finally, the data is input into a suitable software package (such as Prism) for curve fitting analysis. Based on this data, the concentration of the compound required to obtain 50% inhibition effect is obtained by calculation so as to determine the IC₅₀ value.

Table 4 shows the IC₅₀ values (µM) of the representative compounds of the present invention (i.e., the compounds of Examples 4, 9 and 11) in the above biological experiments, wherein Osimertinib (AZD9291) as a control compound is synthesized according to the method described in the following literature:
Discovery of a Potent and Selective EGFR Inhibitor (AZD9291) of Both Sensitizing and T790M Resistance Mutations That Spares the Wild Type Form of the Receptor, J. Med. Chem., 2014, 57 (20), pp 8249-8267.

**Table 4**

| Example No. | Compound No. | IC₅₀ value of Test 4 | IC₅₀ value of Test 5 | Inhibitory selectivity of the compound, Ratio of IC₅₀ value of Test 4 to IC₅₀ value of Test 5 |
|---|---|---|---|---|
| 4 | Compound 11 | >10 | 0.0034 | >2941.18 |
| 9 | Compound 18 | >10 | 0.0052 | > 1923.08 |
| 11 | Compound 20 | >10 | 0.0054 | > 1851.85 |
| 12 | Compound 26 | >10 | 0.0037 | >2702.70 |
| 12 | Compound 27 | >10 | 0.0032 | >3125.00 |
| 12 | Compound 28 | >10 | 0.0040 | >2500.00 |
| 13 | Compound 29 | >10 | 0.0045 | >2222.22 |
| Osimertinib (AZD9291) | | 3.116 | 0.0071 | 438.87 |

As shown in Table 4, the compound of the present invention has a relatively smaller IC₅₀ value than that of Osimertinib (AZD9291) in the proliferation test of BalF3-DTM-EGFR cells. This shows that the compound of the present invention may inhibit proliferation of Ba/F3-DTM-EGFR cells better than Osimertinib (AZD9291). In addition, as shown in Table 4, the right-most column of the table shows the ratio of the IC₅₀ value of Test 4 to that of Test 5, which characterizes the selectivity of the compound on inhibitory effect of BalF3-CAT-EGFR cell proliferation and Ba/F3-DTM-EGFR cell proliferation. A larger value indicates that the compound has better selectivity for inhibition of Ba/F3-DTM-EGFR cell proliferation among BalF3 cells. As shown in Table 4, the selectivity of the inhibitory effect of the compound according to the present invention on Ba/F3-DTM-EGFR cell proliferation is much better than that of Osimertinib (AZD9291).

### Test 6: Test of Plasma Protein Binding Rate

The test of plasma protein binding rate is performed by using the 96-well equilibrium dialysis device from HTDialysis Company. The human plasma samples or mouse plasma samples with a concentration of 2 µM for the test and control compounds are performed equilibrium dialysis at 37°C for 4 hours so as to conduct the protein binding study. 150 µL of plasma sample with the test compound is added to the administration end of each dialysis well, and 150 µL of blank dialysis buffer is added to the receiving end corresponding to the dialysis well. The dialysis plate is then placed in a humidified incubator with 5% CO₂ to incubate at 37°C for 4 hours. After dialysis, 50 µL of the dialyzed buffer sample and the dialyzed plasma sample are transferred to a new 96-well plate (sample receiving plate). The corresponding volume of corresponding blank plasma or buffer is added to the sample. All samples are analyzed by LC/MS/MS after protein precipitation. The concentration of the sample is expressed as the ratio of the peak area of the compound to the internal standard substance (semi-quantitative). Finally, the % Unbound, % Bound, and % Recovery of the compound are calculated according to the formula.

**Table 5**

| Example No. | Compound No. | % Unbound of human plasma sample in Test 6 | % Unbound of mouse plasma sample in Test 6 |
|---|---|---|---|
| 4 | 11 | 10.7 | 7.7 |
| Osimertinib (AZD9291) | | 2.5 | 0.5 |

Under normal circumstances, various drugs are bound to plasma proteins at a certain ratio, and there are often both bound and unbound (free) types in plasma, and only unbound drugs have drug activity. As shown in Table 5, for the plasma protein binding rate test, the compound of the present invention has a relatively larger unbound drug ratio in human and mouse plasma than that of Osimertinib (AZD9291), indicating that the compound of the present invention may increase its unbound drug ratio (i.e., the active drug ratio) in plasma relative to Osimertinib (AZD9291).

The dimensions and values disclosed herein are not intended to be understood as being strictly limited to the precise values stated. Instead, unless otherwise indicated, each such dimension refers to the cited value and the functionally equivalent range surrounding that value.

## Claims

1. A compound of formula (I) or a pharmaceutically acceptable salt thereof: 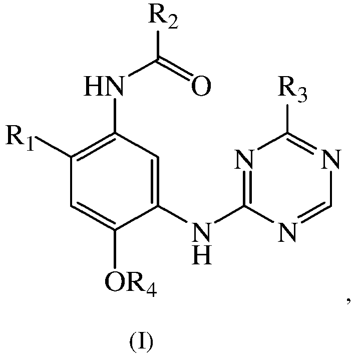
wherein R₁ is -NR₅R₆, and R₅ and R₆ are each independently selected from C₁-C₆ alkyl, and wherein the alkyl is unsubstituted or substituted by -NR₇R₈;
wherein R₂ is selected from C₂-C₆ alkenyl, the alkenyl is unsubstituted or substituted by -NR₇R₈;
wherein at each occurrence R₇ and R₈ are each independently selected from H or C₁-C₆ alkyl; wherein R₃ is selected from: 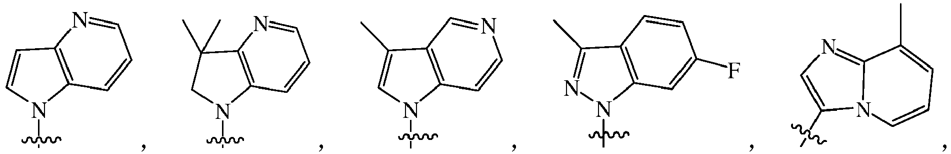 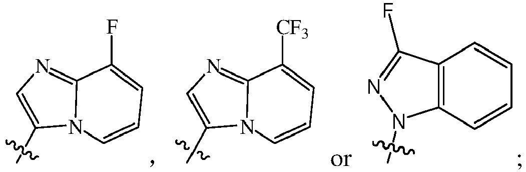 and
wherein R₄ is selected from C₁-C₆ alkyl.

2. The compound or a pharmaceutically acceptable salt thereof according to aclaim 1, wherein the R₁ is selected from:

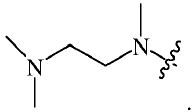

3. The compound or a pharmaceutically acceptable salt thereof according to any one of claims 1-2, wherein the R₂ is selected from:

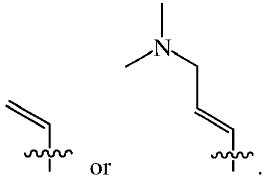

4. The compound or a pharmaceutically acceptable salt thereof according to claim 1, wherein the compound is selected from:

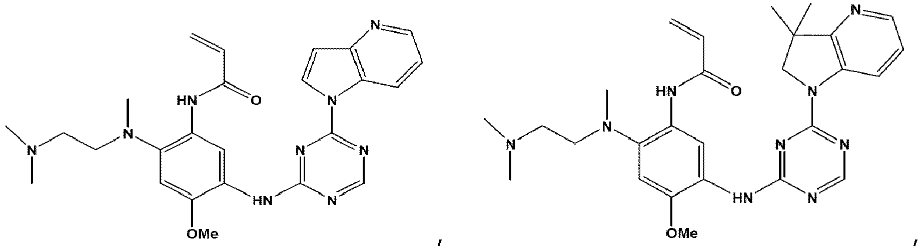

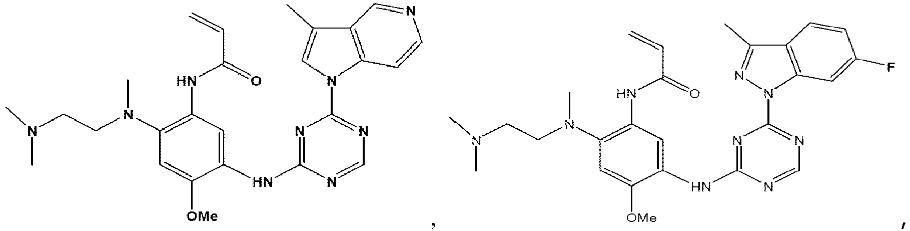

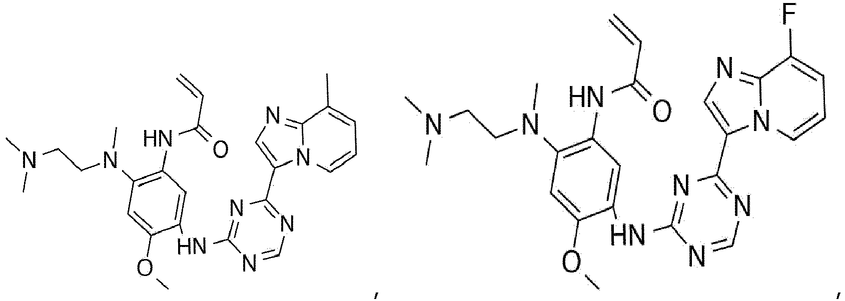

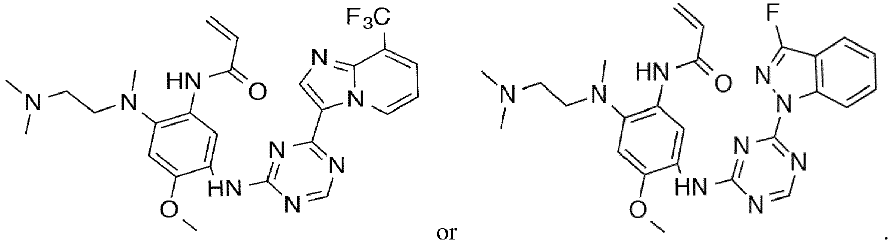

5. A pharmaceutical composition, comprising the compound or a pharmaceutically acceptable salt thereof according to any one of claims 1-4.

6. The compound or a pharmaceutically acceptable salt thereof according to any one of claims 1-4 for use for treating non-small cell lung cancer.

## Patentansprüche

1. Verbindung einer Formel (I) oder pharmazeutisch annehmbares Salz davon: 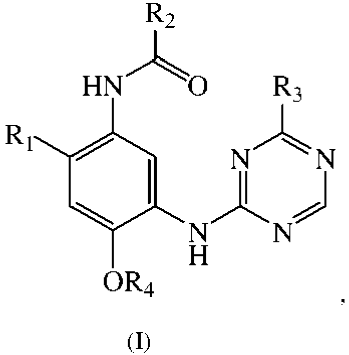
wobei R₁ -NR₅R₆ ist und R₅ und R₆ jeweils unabhängig aus C₁-C₆-Alkyl ausgewählt sind und worin das Alkyl unsubstituiert oder durch -NR₇R₈ substituiert ist;
wobei R₂ ausgewählt ist aus C₂-C₆-Alkenyl, wobei das Alkenyl unsubstituiert oder durch -NR₇R₈ substituiert ist;
wobei bei jedem Auftreten R₇ und R₈ jeweils unabhängig aus H oder C₁-C₆-Alkyl ausgewählt sind;
wobei R₃ ausgewählt ist aus: 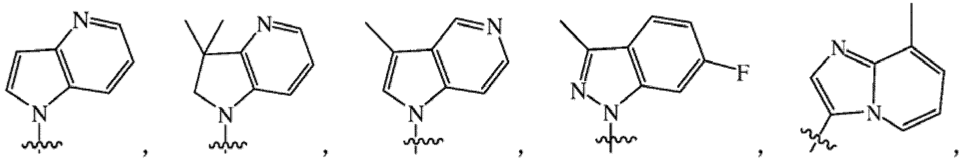 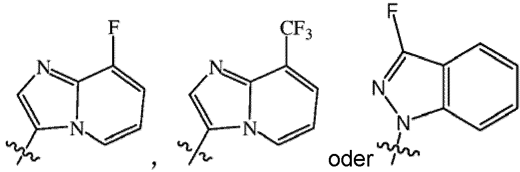 und
Worin R₄ aus C₁-C₆-Alkyl ausgewählt ist.

2. Verbindung oder pharmazeutisch annehmbares Salz davon nach Anspruch 1, wobei R₁ ausgewählt ist aus:

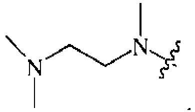

3. Verbindung oder pharmazeutisch annehmbares Salz davon nach einem der Ansprüche 1-2, wobei R₂ ausgewählt ist aus:

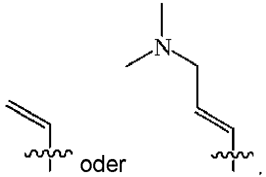

4. Verbindung oder pharmazeutisch annehmbares Salz davon nach Anspruch 1, wobei die Verbindung ausgewählt ist aus:

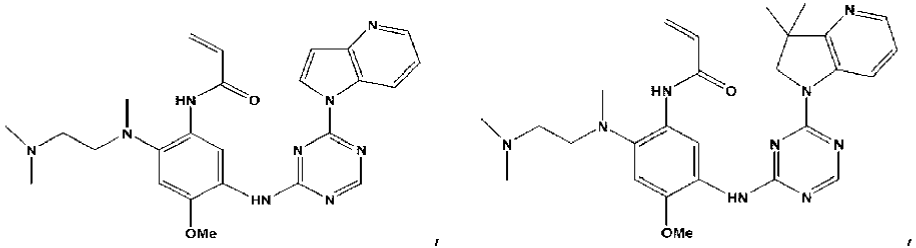

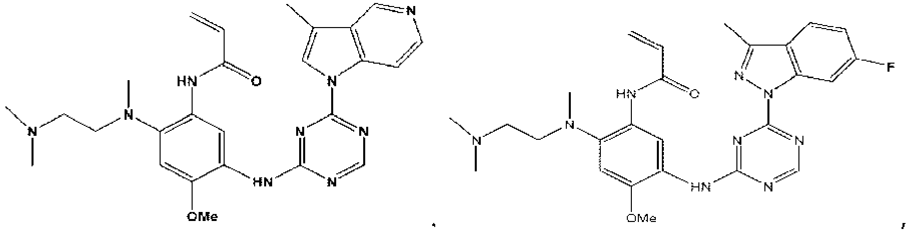

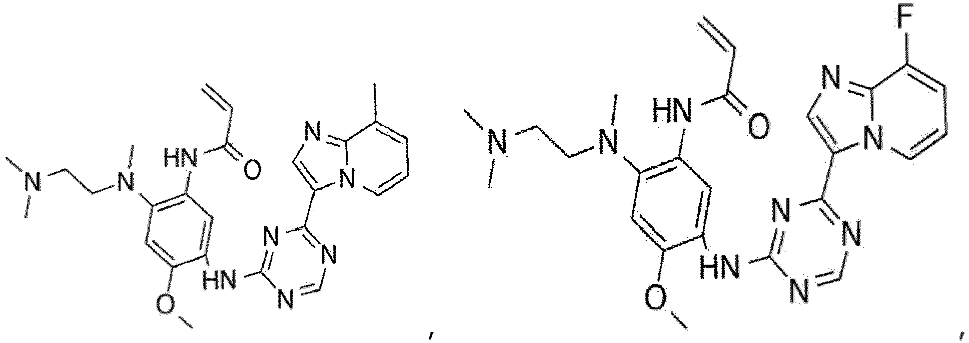

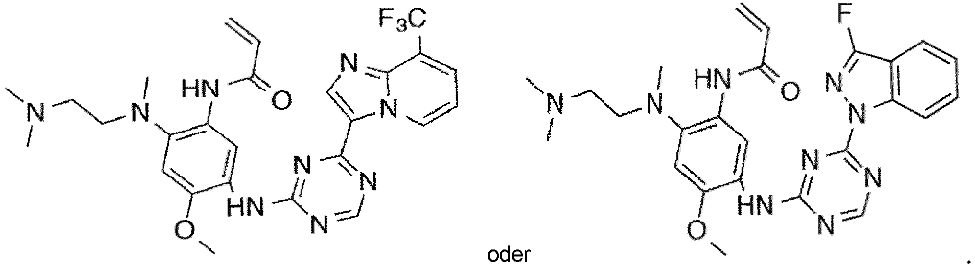

5. Pharmazeutische Zusammensetzung, umfassend die Verbindung oder ein pharmazeutisch annehmbares Salz davon nach einem der Ansprüche 1-4.

6. Verbindung oder pharmazeutisch annehmbares Salz davon nach einem der Ansprüche 1-4 zur Verwendung bei der Behandlung von nicht-kleinzelligem Lungenkrebs.

## Revendications

1. Composé de formule (I) ou son sel pharmaceutiquement acceptable : 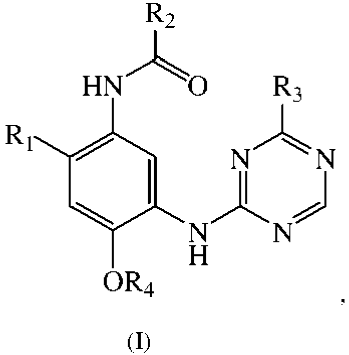
dans lequel R₁ est -NR₅R₆, et R₅ et R₆ sont chacun indépendamment choisis parmi les alkyles en C₁-C₆, et dans lequel l'alkyle est non substitué ou substitué par -NR₇R₈ ;
dans lequel R₂ est choisi parmi les alcényles C₂-C₆, l'alcényle étant non substitué ou substitué par -NR₇R₈ ;
dans lequel, à chaque occurrence, R₇ et R₈ sont choisis indépendamment parmi H ou un alkyle en Ci-Ce ;
dans lequel R₃ est sélectionné parmi : 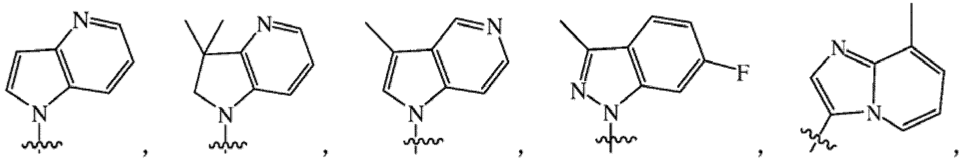 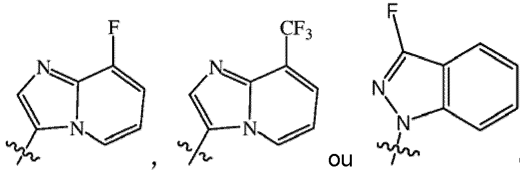 et
dans lequel R₄ est sélectionné parmi un alkyle en C₁-C₆.

2. Composé ou sel pharmaceutiquement acceptable de celui-ci selon la revendication 1, dans lequel le R₁ est choisi parmi :

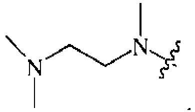

3. Composé ou sel pharmaceutiquement acceptable de celui-ci selon l'une quelconque des revendications 1 à 2, dans lequel le R₂ est choisi parmi :

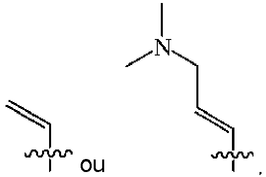

4. Composé ou sel pharmaceutiquement acceptable de celui-ci selon la revendication 1, dans lequel le composé est choisi parmi :

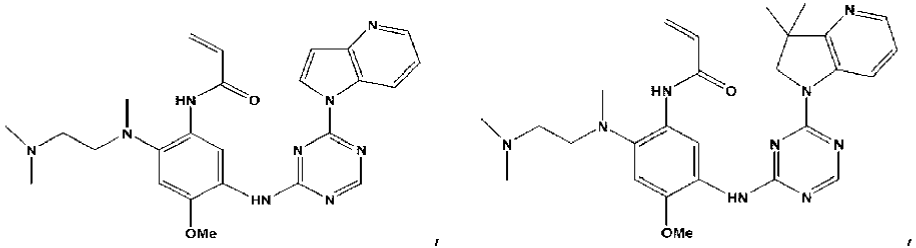

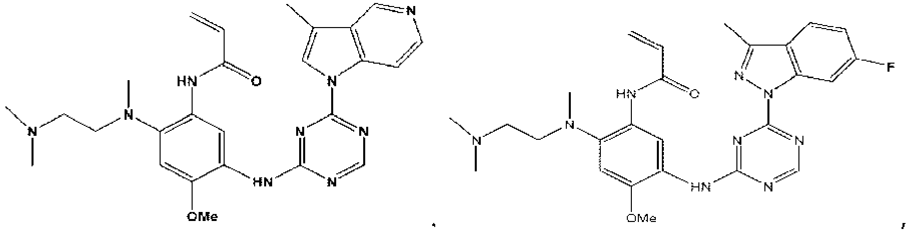

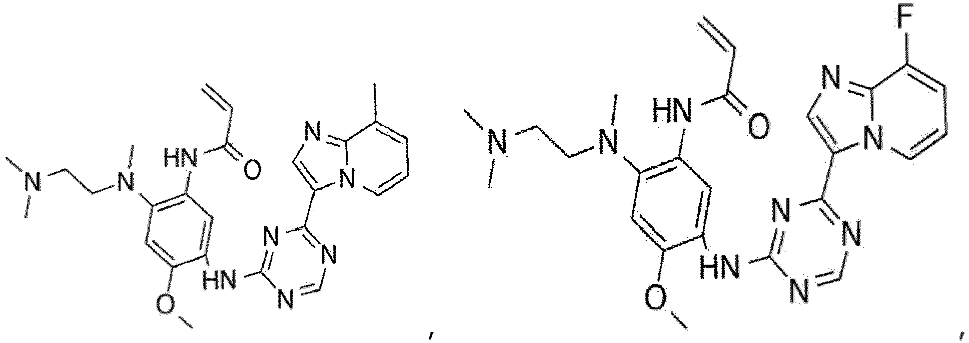

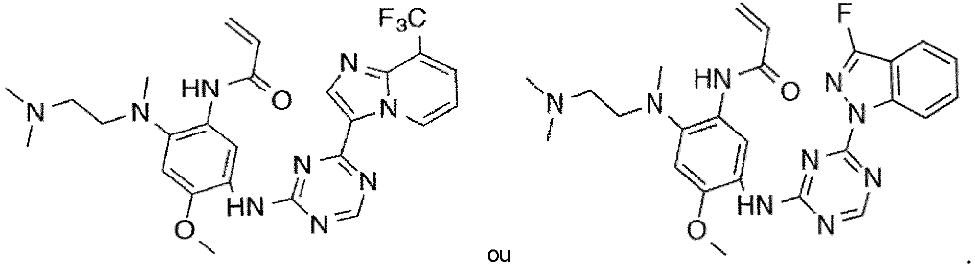

5. Composition pharmaceutique comprenant au moins un composé ou un sel pharmaceutiquement acceptable de celui-ci selon l'une quelconque des revendications 1 à 4.

6. Composé ou un sel pharmaceutiquement acceptable de celui-ci selon l'une quelconque des revendications 1 à 4 pour une utilisation dans le traitement du cancer du poumon non à petites cellules.
